# EUROPEAN PATENT APPLICATION

(11) **EP 1 331 483 A1**
(43) Date of publication of application: **30.07.2003**
(21) Application number: 01982715.3
(22) Date of filing: 31.10.2001
(51) Int. Cl.: G01N 33/574, G01N 33/53

(54) **AGENT FOR DETECTING CANCER'S ABILITY TO METASTASIZE**

(30) Priority: 01.11.2000 JP 2000335077
(71) Applicant: Galpharma Co., Ltd., Takamatsu-shi, Kagawa 761-0301 (JP)
(72) Inventor: HIRASHIMA, Mitsuomi, Takamatsu-shi, Kagawa 761-0322 (JP); YAMAUCHI, Akira, Takamatsu-shi, Kagawa 761-0113 (JP); KAGESHITA, Toshiro, Kumamoto-shi, Kumamoto 862-0951 (JP); NAKAMURA, Takanori, Takamatsu-shi, Kagawa 761-0104 (JP); NISHI, Nozomu, K ita-gun, Kagawa 761-0703 (JP)
(74) Representative: von Kreisler, Alek, Dipl.-Chem.
(86) International application number: JP0109561
(87) International publication number: WO02037114

(57) **Abstract**

By disclosing part of metastasis mechanisms in cancer cells, the metastatic potential of cancer cells will be determinable. Anti-galectin-9 Ab against galectin-9 is contained as an effective component with or without others, thereby enabling the evaluation or detection of galectin-9 expression levels in cancer cells wherein the metastatic potential of the cancer cells can be assessed or detected.

## Description

### Field of the Invention

The present invention relates to a predicting agent for metastasis, an assay for predicting the metastasis, and a prediction system for the metastasis, which enable evaluation or assessment as to whether or not cancer cells have metastatic potential (or matastasizing ability) and how much metastatic potential level the cancer cells have.

The present invention relates to the use of galectin-9 and a galectin-9 expression gene as markers for cancer metastatic potential or as tumor markers, measurement and/or prediction reagents, measurement and/or prediction methods (assays) related to such applications, and measurement and/or prediction systems to be used for such measurement and/or prediction methods (assays).

### Background of the Invention

Cancer cells not only have vigorous proliferation potential but also have infiltration potential that enable cancer cells to destroy tissue on their own and infiltrate into neighboring tissue as well as into blood vessels and lymph vessels. Cancer cells also have metastatic potential to flow along with blood or lymph and metastasize to distant tissue. Such metastasis of cancer cells is a factor that gives rise to unfavorable symptoms in the final stage. Research on the metastasis of cancer has thus been conducted since priorly. In cancer metastasis research from 1992 to 1993, a relationship between a high degree of metastasis and a low expression or inactivation of cadherins, which are cellular adhesion factors, has been reported. Among cadherins, E-cadherin is known as a Ca-dependent intercellular adhesion molecule that is expressed in epithelial cells and has been analyzed for the purpose of clarifying cancer metastasis mechanisms. However, the mechanisms of cancer metastasis could not be explained adequately by E-cadherin alone and it has been indicated that other factors are deeply involved. There have however not been any reports on specific factors involved in cancer metastasis and it has been difficult to predict cancer metastasis accurately. In other words, up until now, the metastatic potential of cancer cells could not be judged at high precision.

### Summary of the Invention

An object of the present invention is to disclose an aspect of the metastasis mechanisms in cancer cells and thereby provide cancer metastatic potential-predicting (or detecting) and/or assessing agents, cancer metastatic potential-detection (or prediction) and/or assessment methods (assays), and prediction (or detection) and/or assessment systems for the cancer metastatic potential (or metastatic property), which enable prediction, judgment or determination of the metastatic potential for cancer cells. In order to achieve this object, the present inventors have carried out an extensive research.

As a result, the present inventors have succeeded in disclosingj that galectin-9 is deeply involved with the metastatic potential of cancer cells (or cancer cell matastatic properties) and that the metastatic potential of cancer cells can be detected or assessed with high precision by measurement or assay of galectin-9 expression in the cancer cells and have thereby come to complete the present invention.

The present invention provides cancer metastatic potential-predicting agents (or cancer metastatic potential-detecting agents) comprising, as an effective component thereof:
(1) an anti-galectin-9 antibody (anti-galectin-9 Ab) against galectin-9; or
(2) a nucleic acid, which hybridizes with a nucleic acid coding for galectin-9 or a constituent domain thereof.
   The cancer cells herein are not restricted to particular species as long as they are detectable in view of galectin-9 or a galectin-9 expression gene and/or the correlation between galectin-9 or a galectin-9 expression gene and the cancer metastatic potential (or cancer metastatic properties) can be disclosed or assessed. Examples of such cancers include malignant tumors of various organs and tissues, such as mammary cancer cells and melanoma cells. The cancer metastatic detection agent according to the present invention is especially favorable for the detection or determination of the metastatic potential (and/or metastatic porperties) of mammary cancer cells and melanoma cells. The present invention also provides cancer metastatic potential measurement and/or detection methods (assays), which comprise measuring, detecting, or quantitatively assaying galectin-9 or a galectin-9 expression gene in a test sample wherein cancer metastatic potential (and/or cancer metastatic porperties) is (are) detected or assessed, relying on the measurement and/or detection (assay) results, and systems (including a set of reagents) to be used for such a detection or assay. These systems may include part or all of the reagents required for cancer metastatic potential detection as a set, may furthermore have attached documents that explain the protocols for the cancer metastatic potential detection or assay, may be a device or equipment, such as a DNA array, etc., and this interpretation shall hold throughout this Specification. It shall be understood that this inventive cancer metastatic potential detection agent, cancer metastatic detection or assay, and system used for the detection or assay are also useful as a prognostic cancer metastatic potential detection tool, cancer metastatic detection or assay, and system used for the detection or assay.
   This invention provides a cancer metastatic potential prediction (or detection) agent or tumor detection and/or measurement agent, a cancer metastatic potential prediction (or detection) or assay, or tumor detection and/or measurement (or assay), and a cancer metastatic prediction (or detection) or tumor detection and/or measurement reagent set or system that are characterized in that galectin-9 or a galectin-9 expression gene is employed as a cancer metastatic potential marker (or cancer metastatic potential predictor) or tumor marker. The present invention also provides a cancer metastatic potential prediction (or detection) agent (cancer metastatic potential predictor) or tumor detection and/or measurement agent, a cancer metastatic potential prediction (or detection) or assay, or tumor detection and/or measurement (or assay), and a cancer metastatic detection or tumor detection and/or measurement reagent set or system that are characterized in that galectin-9 or a galectin-9 expression gene is employed as a prognostic cancer metastatic potential marker or a tumor marker.
   The above objectives and other objectives, features, advantages, and aspects of the present invention are readily apparent to those skilled in the art from the following disclosures. It should be understood, however, that the description of the specification including the following best modes of carrying out the invention, examples, etc. is illustrating preferred embodiments of the present invention and given only for explanation thereof. It will become apparent to the skilled in the art that a great number of variations and/or alterations (or modifications) of this invention may be made based on knowledge from the disclosure in the following parts and other parts of the specification without departing from the spirit and scope thereof as disclosed herein. All of the patent publications and reference documents cited herein for illustrative purposes are hereby incorporated by reference into the present disclosure.

### Brief Description of the Drawings

Fig. 1 is a diagram, showing the conditions and statistical examination results for the relationship between galectin-9 expression and lymphatic metastasis.
Fig. 2 is a diagram, showing the conditions and statistical examination results for the relationship between galectin-9 expression and postoperative recurrence.
Fig. 3 is a diagram, showing the conditions and statistical examination results for the relationship between galectin-9 expression and cases of death.
Fig. 4 is a characteristics diagram, showing the relationship between galectin-9 expression and non-metastatic cumulative survival rate.
Fig. 5 is an electrophoretogram, showing the results of detection of galectin-9 expression by a Western blotting method for high expression lines and low expression lines.
Fig. 6 is a microgram, showing the culturing conditions of a high galectin-9 expression line.
Fig. 7 is a microgram, showing the culturing conditions of a low galectin-9 expression line.

### Best Modes of Carrying out the Invention

The inventive cancer metastatic potential prediction (or detection) agents, cancer metastatic detections or assays, systems used for the detection or assay, and inventive techniques utilizing, as a cancer metastatic potential marker or tumor marker, galectin-9 or a galectin-9 expression gene shall now be described herein below in detail.

A tumor that may metastasize to several sites is a malignant neoplasm, and the term "malignant neoplasm" is generally referred to as being epithelial or non-epithelial and may be distinguished as being cancer, sarcoma, or leukemia, etc. Among the general public, when the neoplasm or tumor is simply called "cancer", it refers to a malignant neoplasm or tumor. As used herein, the term "cancer" is employed in the broadest sense and should not be interpreted as being just an epithelial malignant neoplasm. The term "cancer" used herein may cover epithelial malignant tumors and non-epithelial malignant tumors (including those that are tumorigenic and non-tumorigenic), such as skin cancers (which may include melanomas), mammary cancers, ovarian cancers, uterine cancers, malignant testicular tumors, prostatic cancers, urinary bladder cancers, renal cancers, thyroid cancers, pharyngeal/larynx cancers, lingual cancers, maxillary cancers, esophageal cancers, stomach cancers, colon/rectal cancers, lung/bronchial cancers, liver cancers (including liver cell cancers and intrahepatic bile duct cancers), extrahepatic bile duct/gall bladder cancers, pancreatic cancers, leukemia, malignant lymphoma, plasmacytoma, osteosarcoma, chondrosarcoma, leiomyosarcoma, rhabdomyosarcoma, liposarcoma, fibrosarcoma, malignant hemangioma, malignant hemangioendothelioma, brain tumors (including meningioma, glyoma, astrocytoma), etc., but is not restricted to these. It shall be understood as not being restricted to particular species as long as galectin-9 can be detected and its correlation with cancer metastatic potential can be disclosed or uncovered.

In one mode, this inventive cancer metastatic potential prediction (or detection) agent contains an anti-galectin-9 antibody as an effective element. Herein, the anti-galectin-9 component may be obtained as a polyclonal or monoclonal antibody.

As used herein, the term "antibody" is used in the broadest sense and may cover a single species of desirable monoclonal antibodies against any of galectin-9 polypeptides or related peptide fragments thereof and antibody compositions having a specificity to various epitopes thereof, further monovalent or polyvalent antibodies and polyclonal and monoclonal antibodies, and also those which are intact molecules or fragments and derivatives thereof, including F(ab')₂, Fab' and Fab fragments, and also chimeric antibodies, hybrid antibodies each having at least two antigen or epitope binding sites, or bispecific recombinant antibodies (e.g., quadromes, triomes, etc.), interspecies hybrid antibodies, anti-idiotypic antibodies and those which have been chemically modified or processed and must be regarded as derivatives of these antibodies and further which may be produced either by adopting cell fusion or hybridoma techniques or antibody engineering or by using synthetical or semisynthetical techniques in known manner, which may be prepared either by the known conventional methods in view of antibody production or by recombinant DNA techniques, and which have neutralizing or binding properties with respect to the target antigen substances or target epitopes described and defined herein. Preferably the inventive antibodies are especially those capable of specifically identifying or distinguishing intact (naturally-occurring type) galectin-9M (galectin-9 medium isoform or medium type galectin-9) polypeptide or intact (naturally-occurring type) galectin-9L (galectin-9 long isoform or long type galectin-9) polypeptide, for example, those which specifically recognize any of galectin-9L and galectin-9M polypeptides distinguishably from galectin-9S (galectin-9 short isoform or short type galectin-9).

In order to obtain an anti-galectin-9 antibody as a polyclonal antibody, a mammal, bird, etc. is immunized with an immunogen, galectin-9, or a fragment thereof, that is, a peptide that is a fragment of the galectin-9 sequence, and an antiserum is collected from the immunized mammal, bird, etc. The polyclonal antibodies contained in this antiserum may then be used.

Though a mammal immunized with galectin-9 as the sensitizing antigen is not restricted to particular species, but includes, in general, rodents, such as mouse, rat, hamster, etc., or rabbit, lamb, goat, cattle, horse, pig, dog, cat, or monkey or other primates, or birds, such as chicken. In some cases, it is preferable to select the animal in consideration of the compatibility with the parent cell to be used for cell fusion.

The immunization of an animal with the sensitizing antigen is carried out according to known methods. For example, as a general method, the sensitizing antigen is injected peritoneally or subcutaneously into a mammal, etc. A suitable carrier may also be used for immunization with the sensitizing antigen.

The immunized animal is kept for a predetermined period and the antiserum containing the polyclonal antibody may then be prepared from blood collected from the animal. The antiserum thus obtained is used as a cancer metastatic detecting agent after checking that the antiserum specifically recognizes galectin-9.

First, galectin-9 used as the sensitizing antigen for acquiring the antibodies may be obtained by expression of a known galectin-9 gene/galectin-9 amino acid sequence. The amino acid sequence of galectin-9L is shown as SEQ ID NO: 1, the amino acid sequence of galectin-9M is shown as SEQ ID NO: 2, and the amino acid sequence of galectin-9S is shown as SEQ ID NO: 3. That is, after a gene sequence encoding galectin-9 or a partial domain thereof, a partial protein or peptide fragment of galectin-9, or a peptide having a partial amino acid sequence corresponding to the amino acid sequence of galectin-9, is inserted into a known expression vector with which a suitable host cell is transformed or transfected, and targeted galectin-9 proteins or a partial domain protein thereof, partial protein or polypeptide fragment of galectin-9, or a peptide having the partial amino acid sequence corresponding to the amino acid sequence of galectin-9 is isolated and/or purified from the transformed or transfected host cell or its culture medium with known methods.

Gene recombination techniques (including recombinant DNA techniques) may be carried out in accordance to methods described in, for example, J. Sambrook, E. F. Fritsch & T. Maniatis, "Molecular Cloning: A Laboratory Manual (2nd edition)", Cold Spring Harbor Laboratory Press, Cold Spring Harbor, New York (1989); D. M. Glover et al. ed., "DNA Cloning", 2nd ed., Vol. 1 to 4, (The Practical Approach Series), IRL Press, Oxford University Press (1995); The Japanese Biochemical Society ed., "Zoku Seikagaku Jikken Koza 1, Idenshi Kenkyuho II" (Supplementary Lectures on Biochemical Laboratory Techniques 1, Genetic Research Methods II), Tokyo Kagaku Dojin (1986); The Japanese Biochemical Society ed., "Shin Seikagaku Jikken Koza 2, Kakusan III (Kumikae DNA Gijutsu)" (New Lectures on Biochemical Laboratory Techniques 2, Nucleic Acids III (Recombinant DNA Techniques)), Tokyo Kagaku Dojin (1992); R. Wu ed., "Methods in Enzymology", Vol. 68 (Recombinant DNA), Academic Press, New York (1980); R. Wu et al. ed., "Methods in Enzymology", Vol. 100 (Recombinant DNA, Part B) & 101 (Recombinant DNA, Part C), Academic Press, New York (1983); R. Wu et al. ed., "Methods in Enzymology", Vol. 153 (Recombinant DNA, Part D), 154 (Recombinant DNA, Part E) & 155 (Recombinant DNA, Part F), Academic Press, New York (1987); J. H. Miller ed., "Methods in Enzymology", Vol. 204, Academic Press, New York (1991); R. Wu et al. ed., "Methods in Enzymology", Vol. 218, Academic Press, New York (1993); S. Weissman (ed.), "Methods in Enzymology", Vol. 303, Academic Press, New York (1999); J. C. Glorioso et al. (ed.), "Methods in Enzymology", Vol. 306, Academic Press, New York (1999) and others, as well as methods indicated in references quoted in the above documents and substantially equivalent or modified methods, the disclosures of which are incorporated herein by reference (hereinafter, all such techniques or methods shall be referred to as "gene recombination techniques").

Nucleic acids encoding galectin-9, a constituent domain thereof, or a fragment thereof may include any species as long as they have a nucleotide base sequence with the same efficacy as coding for a peptide exerting antigenicity or other biological activities substantially equivalent to galectin-9. The galetic-9-encoding nucleic acid may be single-strand DNA, double-strand DNA, RNA, DNA:RNA hybrids, synthetic DNA, etc. The galetic-9 nucleic acid may be any of human genome DNA, human genomic DNA libraries, cDNA derived from human tissues or cells, and synthetic DNA. The nucleotide sequence of the galectin-9 coding nucleic acid may be modified (for example, added, removed, substituted, etc.) and may contain such modifications. The nucleic acid may also be a naturally-occurring variant. Such nucleic acids may are those coding for a galectin-9L peptide or a part thereof and preferably DNA. The term "nucleotide base sequence with the same efficacy" include nucleotide base sequences that hybridize with 5 or more continuous bases, preferably 10 or more continuous bases, more preferably 15 or more bases, and even more preferably 20 or more bases among base sequences coding for the amino acid sequence of SEQ ID NO: 1 in the Sequence Listing under stringent conditions and encode an amino acid sequence substantially equivalent to galectin-9 and complementary chains of such base sequences.

As used herein, the anti-galectin-9 antibody may include mammal-derived monoclonal antibodies.

Monoclonal antibodies prepared against antigenic substances are produced by any method capable of providing production of antibody molecules by a series of cell lines in culture. The modifier "monoclonal" indicates the character of the antibody as being obtained from a substantially homogeneous population of antibodies, and is not to be construed as requiring production of the antibody by any particular method. The individual amtibodies are those containing a population of identical antibodies except for possible naturally-occurring mutations that may be present in minor amounts. Monoclonal antibodies are highly specific, being directed against a single antigenic site. In contrast to conventional (polyclonal) antibody preparations which typically include different antibodies directed against different determinants (epitopes), each monoclonal antibody is directed against a single determinant on the antigen. In addition to their specificity, the monoclonal antibodies are advantageous in that they are synthesized by the hybridoma culture, uncontaminated or little contaminated by other immjunoglobulins. The monoclonal antibodies included within the scope of the invention include hybrid and recombinant antibodies. They are obtainable by substituting a constant domain of an antibody for a variable domain (e.g., "humanized" antibodies), or a heavy chain for a light chain, by substituting a chain from one species with a chain from another species, or by fusing to heterogeneous proteins, regardless of species of origin or immunoglobulin class or subclass designation, so long as they exhibit the desired biological activity (e.g., U.S. Pat. No. 4,816,567; Monoclonal Antibody Production Techniques and Applications, pp.79 to 97, Marcel Dekker, Inc., New York, 1987; etc.).

Preferable techniques for producing monoclonal antibodies include, for example, the methods using hybridoma cells (G. Kohler and C. Milstein, Nature, 256, pp.495-497 (1975)); the methods using human B cell hybridomas (Kozbor et al., Immunology Today, 4, pp.72-79 (1983); Kozbor, J. Immunol., 133, pp.3001 (1984); Brodeur et al., Monoclonal Antibody Production Techniques and Applications, pp.51-63, Marcel Dekker, Inc., New York (1987); triome methods; EBV-hybridoma methods (Cole et al., Monoclonal Antibodies and Cancer Therapy, Alan R. Liss, Inc., pp.77-96 (1985))(techniques for production of human monoclonal antibodies); U.S. Pat. No. 4,946,778 (techniques for production of single-chain antibodies), as well as the following documents:
S. Biocca et al., EMBO J, 9, pp.101-108 (1990); R.E. Bird et al., Science, 242, pp.423-426 (1988); M.A. Boss et al., Nucl. Acids Res., 12, pp.3791-3806 (1984); J. Bukovsky et al., Hybridoma, 6, pp.219-228 (1987); M. DAINO et al., Anal. Biochem., 166, pp.223-229 (1987); J.S. Huston et al., Proc. Natl. Acad. Sci. USA, 85, pp.5879-5883 (1988); P.T. Jones et al., Nature, 321, pp.522-525 (1986); J.J. Langone et al. (ed.), "Methods in Enzymology", Vol. 121 (Immunochemical Techniques, Part I: Hybridoma Technology and Monoclonal Antibodies), Academic Press, New York (1986); S. Morrison et al., Proc. Natl. Acad. Sci. USA, 81, pp.6851-6855 (1984); V.T. Oi et al., BioTechniques, 4, pp.214-221 (1986); L. Riechmann et al., Nature, 332, pp.323-327 (1988); A. Tramontano et al., Proc. Natl. Acad. Sci. USA, 83, pp.6736-6740 (1986); C. Wood et al., Nature, 314, pp.446-449 (1985); Nature, 314, pp.452-454 (1985), references quoted in the above documents (the the disclosures of which are incorporated herein by reference).

The monoclonal antibodies herein specidically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they have the desirable biological activity (U.S. Pat. No. 4,816,567; Morrison et al., Proc. Natl. Acad. Sci. USA, 81, pp.6851-6855 (1984)).

The monoclonal antibodies of this invention may be produced from mammalian origin-derived hybridoma or may be produced from a host that has been transformed by an expression vector containing an antibody gene according to genetic engineering techniques.

A monoclonal antibody producing hybridoma that produces anti-galectin-9 Ab may be prepared by cell fusion techniques utilizing myeloma cells as follows:

That is, such a hybridoma may be prepared by performing immunization using galectin-9 or a fragment thereof as the sensitizing agent in accordance with a normal immunization method, fusing the immune cells obtained with known parent cells in accordance with a normal cell fusion method, and screening the monoclonal antibody producing cells by a normal screening method. The method of preparing galectin-9 or fragments thereof, the method of immunizing a mammal, etc. may be carried out in accordance with the above-described techniques for preparing an antiserum containing polyclonal antibodies. In particular in this case, after immunization of mammals, immune cells are taken out from a mammal wherein an increased level of desired antibodies has be en confirmed for the serum and subjected to cell fusion. Particularly preferable immune cells are spleen cells.

Mammal myeloma cells are used as the other parent cells to be fused with the abovementioned immune cells. Any of various known cell lines may be used for the myeloma cells. The cell fusion between the immune cells and myeloma cells may basically be carried out in accordance with a known method, such as Kohler and Milstein's method (Kohler, G. and Milstein, C., Methods Enzymol. (1981) 73, 3-46), etc.

Described herein below is the production of antibodies, including embodiments of monoclonal antibodies.

The monoclonal antibody to be used in the present invention may be a monoclonal antibody obtained by utilizing cell fusion techniques with myeloma cells. The monoclonal antibody to be used in the present invention can be produced by the following processes: (1) preparation of immunogenic antigens; (2) immunization of animals with immunogenic antigens; (3) preparation of myeloma cells; (4) cell fusion between antibody-producing cells and myeloma cells; (5) selection and cloning of hybridomas (hybrid cells), and (6) production of monoclonal antibodies.

(1) The preparation of immunogenic antigens may be carried out as follows:

The antigen as used herein includes, as disclosed above, isolated native galectin-9L polypeptides or fragments derived therefrom (which include part of such proteins, i.e., domain polypeptides, link polypeptides, fragments, partial peptides, and synthetic polypeptides, and may include native galectin-9M polypeptide). The antigen also includes a suitable oligopeptide chemically synthesized based on information on the determined amino acid sequence of galectin-9.
Representative examples thereof are peptides having at least five continuous amino acids among the amino acid residues existing in a region selected from the group consisting of (1) amino acid sequences of SEQ ID NO: 1 and 2 in the Sequence Listing or amino acid sequences of constituent domains thereof (part of such sequences); (2) amino acid sequences of constituent domains for SEQ ID NO: 3; (3) amino acid sequences of SEQ ID NO: 4, 5, 7, 8, and 9 of the Sequence Listing;
(4) among SEQ ID NO: 1 of the Sequence Listing, constituent amino acid sequences for the C-terminal domain following the amino acid sequence corresponding to SEQ ID NO: 4 or partial fragments thereof; and (5) among SEQ ID NO: 1 of the Sequence Listing, constituent amino acid sequences for the N-terminal domain preceding the amino acid sequence corresponding to SEQ ID NO: 4 or partial fragments thereof.

Although the antigen may be used to immunize animals after being mixed with a suitable adjuvant without any modifications, it can be used after formation of immunogenic conjugates. For example, the antigen to be used for such an immunogen may be a fragment derived from galectin-9 or a synthetic polypeptide fragment obtained via selecting characteristic sequence areas based on the amino acid sequences of galectin-9, designing a polypeptide, and performing chemical synthesis. The fragments may be coupled with any of various carrier proteins via suitable coupling agents to form immunogenic conjugates such as hapten-proteins. The immunogenic conjugates can be used to design monoclonal antibodies that can react with (or recognize) specific sequences exclusively. The polypeptide thus designed has a cysteine residue or the like added in advance so that an immunogenic conjugate may be prepared readily. To couple with a carrier protein or the like, the carrier protein is first activated. This activation may include incorporation of an activated binding group thereinto, etc.

The activated binding groups include (1) active ester or active carboxyl groups such as a nitrophenyl ester group, a pentafluorophenyl ester group, a 1-benzotriazol ester group, and an N-succinimido ester group; (2) active dithio groups such as a 2-pyridyldithio group, etc. The carrier proteins include keyhole limpet haemocyanin (KLH), bovine serum albumin (BSA), ovalbumin, globulin, polypeptides such as polylysine, bacterial components such as BCG or the like.

(2) The immunization of animals with immunogenic antigens may be carried out as follows:

Animals can be immunized according to techniques known to those skilled in the art and, as described in for example, Shigeru Muramatsu et al. ed., "Jikken Seibutsugaku Koza 14, Menekiseibutsugaku" (Lectures on Experimental Biology 14, Immunobiology), Maruzen K. K., Japan (1985); The Japanese Biochemical Society ed., "Zoku Seikagaku Jikken Koza 5, Menekiseikagaku Kenkyuho" (Supplementary Lectures on Biochemical Laboratory Techniques 5, Immunological and biochemical Research Methods), Tokyo Kagaku Dojin, Japan (1986); The Japanese Biochemical Society ed., "Shin Seikagaku Jikken Koza 12, Bunshimenekigaku III, Kogen-Kotai-Hotai" (New Lectures on Biochemical Laboratory Techniques 12, Molecular Immunology III, Antigens-Antibodies-Complements), Tokyo Kagaku Dojin, Japan (1992) , etc. Immunization can be performed in a mammal, for example, by one or more injections of an immunizing agent (and, if desired, with an adjuvant). Typically, this is carried out by multiple subcutaneous or intraperitoneal injections with the immunizing agent and/or adjuvant into the mammal. The immunizing agent may include those containing the aforementioned antigen peptide or its related peptide fragment. The immunizing agent used may be a conjugate coupled with a protein known to be immunogenic in the mammal that will be subjected to immunization. Examples of such immunogenic proteins which may be employed include the aforementioned carrier proteins. The adjuvant includes Freund's complete adjuvant, Ribi adjuvant, pertussis vaccine, BCG, lipid A, liposome, aluminium hydroxide, silica, etc. The Immunization is carried out with suitable animals, including mice such as BALB/c, hamsters, and others. The antigen dose is, for example, approximately 1 to 400 µ g/animal for mice. Generally, the antigen is injected intraperitoneally or subcutaneously into a host animal, which will be additionally immunized by repeated courses wherein intraperitoneal, subcutaneous or intravenous administrations are carried out approximately 2 to 10 times at 1- to 4-week intervals, preferably 1- to 2-week intervals. For immunization, BALB/c mice, as well as F1 mice between BALB/c mice and other line mice, etc. can be used. As required, the animal can be assessed for its immunization level via constructing an antibody titer assay system and measuring the titer of an antibody. The antibody of the present invention may include those obtainable from such immunized animals, for example, anti-serum, polyclonal antibodies, etc.

(3) The preparation of myeloma cells may be carried out as follows:

Immortal cell lines (tumor cell lines) to be used for cell fusion can be selected from non-immunoglobulin-producing cell lines. The cell lines to be used for cell fusion may include, for example, P3-NS-1-Ag4-1 (NS-1, Eur. J. Immunol., 6: 511-519, 1976), SP-2/O-Ag14 (SP-2, Nature, 276: 269-270, 1978), mouse myeloma MOPC-21 cell line-derived P3-X63-Ag8-U1 (P3U1, Curr. topics Microbiol. Immunol., 81: 1-7, 1978), P3-X63-Ag8 (X63, Nature, 256: 495-497, 1975), P3-X63-Ag8-653 (653. J. Immunol., 123: 1548-1550, 1979), etc. Mouse myeloma cell lines (8-azaguanine resistant cell lines) can be sub-cultured in a cell culture medium, such as Dulbecco's modified Eagle's medium (DMEM) and RPMI-1640 medium, supplemented with antibiotics such as penicillin, amikacin or the like, fatal calf serum (FCS) or the like and 8-azaguanine (for example, 5 to 45µ g/ml). The specified number of cell lines can be prepared by passage the normal medium 2 to 5 days prior to cell fusion. The cell lines to be used may be cultured on the normal medium after the frozen and preserved cell lines have been completely thawed at approximately 37°C and have been washed on the normal medium such as RPMI-1640 three or more times, and the specified number of cell lines may be prepared.

(4) The cell fusion between antibody-producing cells and myeloma cells may be carried out as follows:

After animals such as mice are immunized according to the above step (2), their spleens are taken out in two to five days from final immunization, and the spleen cell suspension is obtained. Besides the spleen cells, lymph node cells at various sites of organisms can be obtained and used for cell fusion. To be more specific, the cell fusion is carried out in a normal nutrient culture medium, for example, in the presence of a cell fusion accelerator. The culture medium used for the aforementioned cell fusion include, for example, RPMI 1640 medium and MEM, favorable for the growth of the aforementio ned myeloma cell lines, and other normal cell media used in this cell culture. Furthermore, serum supplements, such as fetal calf serum (FCS), may be used in combination. The spleen cell suspension thus obtained and the myeloma cell lines obtained according to the above step (3) are placed in a culture medium, such as minimum essential medium (MEM), DMEM and RPMI-1640 medium, , followed by addition of a cell fusion accelerator, such as polyethylene glycol (PG). The cell fusion accelerator used may include a variety of other agents known in the relevant fields, such as inactivated HVJ (Hemagglutinating Virus of Japan, "Sendai virus"). For example, 0.5 to 2 ml of 30 to 60% polyethylene glycol may be preferably used. Polyethylene glycol with molecular weights of 1,000 to 8,000 can be used, more preferably polyethylene glycol with molecular weights of 1,000 to 4,000. The preferred concentration of polyethylene glycol in the fusion medium is from 30 to 60%. As required, a small amount of an aid such as dimethyl sulfoxide, may be added to promote fusion efficiency. The usage ratio of immune cells to myeloma cells (that is, spleen cell (lymphocyte) : myeloma cell line ratio) to be used for fusion may be set as suited, and is, for example, preferably 1:1 to 20:1 and more preferably falls within 4:1 to 10:1.

For cell fusion, predetermined amounts of immune cells and myeloma cells are mixed well in a culture medium, into which a solution of PEG (for example, with an average molecular weight of approximately 1,000 to 6,000) preheated to approximately 37°C is added normally at a concentration of 30 to 60% (w/v). The resultant cell mixture was mixed to form targeted fused cells (hybridomas). Thereafter, the cell fusion agent and others, unfavorable for hybridoma growth, are eliminated by repeated operations of successively adding suitable culture media, centrifuging, and removing the supernatant.

The fusion reaction is carried out for 1 to 10 minutes, before the addition of a cell culture medium such as RPMI-1640 medium. Fusion reaction can be done several times. After fusion reaction, cells are separated by centrifugation and the like, and then transferred to a selection medium.

(5) The selection and cloning of hybridomas (hybrid cells) may be carried out as follows:

The selection media include conventionally known "HAT medium", i.e., FCS-containing MEM, RPMI-1640 medium, and other media, which are supplemented with hypoxanthine, aminopterin, and thymidine. Culturing in this HAT medium is continued for a period (normally a few days to a few weeks) adequate for the dying out of cells (non-fused cells), exclusive of targeted hybridomas. The replacement method for the selection medium is to replenish a volume equivalent to the capacity dispensed to the medium plate on the following day, after which the medium is replaced by half an amount with HAT medium every 1 to 3 days. The replacement can be modified depending on situations. Eight to sixteen days after fusion, the medium may be exchanged every 1 to 4 days with conventionally known "HT medium" wherein aminopterin is excluded. As a feeder cell, for example, mouse thymocyte can be used, which is sometimes effective.

The supernatant of a culture well with vigorously growing hybridomas is screened with an assay system such as radioimmunoassay (RIA), enzyme-linked immunosorbent assay (ELISA), fluoroimmunoassay (FIA), luminescence immunoassay (LIA), and Western blotting, or with a fluorescence activated cell sorter (FACS), etc. using a predetermined peptide fragment as an antigen or a labeled anti-mouse antibody to measure target antibodies. The target antibody-producing hybridoma is then cloned. Cloning is carried out by picking up colonies in an agar medium or by the limiting dilution. The limiting dilution is preferred. Cloning should be performed preferably several times. The monoclonal antibody-producing hybridomas thus prepared may be subcultured in a normal culture medium or be subjected to long-term preservation in liquid nitrogen.

(6) The production of monoclonal antibodies may be carried out as follows:

For acquisition of monoclonal antibodies from the hybridoma, the methods employed may include culturing the hybridoma according to usual methods to give the antibody in the form of culture supernatants, implanting and growing the hybridoma in a compatible mammal to harvest the antibody in the form of ascites, etc. The former method is suited for obtaining a highly pure antibody while the latter method is suited for antibody mass production.

The hybridoma cells thus obtained are cultured in a suitable growth medium such as FCS-containing MEM and RPMI-1640 medium, and a desired monoclonal antibody can be obtained from the culture supernatant. Large amounts of monoclonal antibodies can be produced by propagating hybridomas as ascites tumors, etc. In this case, each hybridoma is implanted intraperitoneally in a histocompatible animal isogenic to an animal from which the myeloma cell originated, and in vivo propagated. Or each hybridoma may be inoculated, for example, in nude mice, and in vivo propagated to produce the monoclonal antibody in the ascites of the animals. The produced monoclonal antibody can be recovered from the ascetic fluid. Prior to implantation of hybridomas, the animal is pretreated intraperitoneally with mineral oils such as pristane (2,6,10,14-tetramethylpentadecane). After the pretreatment, the hybridoma can be propagated therein and the ascitic fluid can be harvested. The ascitic fluid may be used as a monoclonal antibody without purification or after purification by conventionally known methods, including salting out such as precipitation with ammonium sulfate, gel filtration with Sephadex and the like, ion exchange chromatography, electrophoresis, dialysis, ultrafiltration, affinity chromatography, high-performance liquid chromatography, etc. The isolated or purified products can be employed as monoclonal antibodies. Preferably, the monoclonal antibody-containing ascitic fluid is fractionated with ammonium sulfate and separated and purified by treatments with anion exchange gel such as DEAE-Sepharose, an affinity column such as protein A column, etc. More preferably, it is treated with affinity chromatography using immobilized antigens or antigen fragments (for example, synthetic peptides, recombinant antigen proteins or peptides, portions which the antibody can specifically recognize, etc.); affinity chromatography with immobilized protein A; hydroxyapatite chromatography; etc.

In addition, it is possible to use transgenic mice and other organisms including other mammals for expressing antibodies such as humanized antibodies against the immunogenic polypeptide products of the present invention.

It is also possible to produce antibodies with recombinant DNA techniques wherein the antibody thus obtained in a large amount is sequenced and/or a nucleotide sequence coding for the antibody obtained from the hybridoma cell line is employed.

Nucleic acids each coding for the monoclonal antibody may, for example, be isolated and sequenced with conventional procedures (e.g., use of oligonucleotide probes capable of binding specifically to genes encoding the heavy or light chain of murine antibodies). Once isolated, the DNA may be placed into expression vectors, which are then transfected into host cells such as CHO cells and COS cells. The DNA may be modified, for example, by substituting the coding sequence for human heavy- and light-chain constant domains for the homologous murine sequences (Morrison et al., Proc. Natl. Acad. Sci. USA, 81: 6581, 1984). Thus, chimeric and hybrid antibodies having a desired binding specificity can be prepared. Further, antibodies can be modified, including preparations of chimeric or hybrid antibodies by adaptations of known methods in synthetic protein chemistry, including those involving coupling agents as listed hereinbelow.

Humanized antibodies are achievable by known techniques in the art (e.g., Jones et al., Nature, 321: pp.522 to 525 (1986); Riechmann et al., Nature, 332: pp.323 to 327 (1988); Verhoeyen et al., Science, 239: pp.1534 to 1536 (1988)).

Human monoclonal antibodies can be achieved according to known techniques in the art. For producing human monoclonal antibodies, human myeloma cells and human-mouse hetero-myeloma cells are known in the art (Kozbor, J. Immunol., 133, pp.3001 (1984); Brodeur et al., Monoclonal Antibody Production Techniques and Applications, pp.51 to 63, Marcel Dekker, Inc., New York (1987)). Methods for making bispecific antibodies are known in the art (Millstein et al., Nature, 305: pp.537 to 539 (1983); WO, A, 93/08829; Traunecker et al., EMBO J., 10: pp.3655 to 3659 (1991); Suresh et al., "Methods in Enzymology", Vol. 121, pp.210 (1986)).

These antibodies may be treated with enzymes such as trypsin, papain, pepsin and others and occasionally be subjected to reduction to produce antibody fragments including Fab, Fab', and F(ab')₂. These antibody fragments may be occasionally used.

The antibodies may be employed in any known assay method, such as competitive binding assays, direct and indirect sandwich assays, and immunoprecipitation assays (Zola, Monoclonal Antibodies: A Manual of Techniques, pp.147 to 158 (CRC Press, Inc., 1987)).

Any method known in the art may be employed for separately conjugating the antibody to the detectable moiety. Such methods include those methods described in David et al., Biochemistry, Vol. 13, pp. 1014-1021 (1974); Pain et al., J. Immunol. Meth., 40: pp. 219-231 (1981); and "Methods in Enzymology", Vol. 184, pp. 138-163 (1990). The antibody to be labeled with a marker may include IgG fractions, and specific binding fragments Fab' obtainable by reduction after pepsin digestion. The labels include enzymes (e.g., peroxidase, alkaline phosphatase, beta-D-galactosidase, etc.), chemical substances, fluorescences, radioisotopes, and the like, as disclosed herein below.

In the present invention, detection (including prediction) and measurement (assay) can be carried out by immunostaining including, for example, staining of tissues and cells, immunoassays including, for example, competitive immunoassay and non-competitive immunoassay, radioimmunoassay (RIA), FIA, LIA, EIA, ELISA, etc. The detection and measurement (assay) can also be carried with or without B-F separation. Preferably, the detection and measurement is carried out preferably by RIA, EIA, FIA, and LIA, as well as sandwich assay. In the sandwich-type assay for example, one of the antibodies is set against this inventive galectin-9L polypeptide or galectin-9L-related peptide fragments and the other against the C-terminal residues of galectin-9 wherein one of both the antibodies is detectably labeled and the other antibody capable of recognizing the same antigen is immobilized on a solid phase (needless to say, other combinations are also possible and may be designed as suitable according to the purpose). As required, incubation is carried out to sequentially react a sample to be assayed, labeled antibodies, and immobilized antibodies. After the non-binding antibodies are separated, the label is detected or measured. The amount of the measured label is proportional to the amount of an antigen, i.e., a galectin-9 polypeptide antigen. This assay is referred to as simultaneous sandwich assay, forward sandwich assay, or reverse-sandwich assay, based on the difference according to the addition sequence of the insolubilized antibody and the labeled antibody. For example, washing, stirring, shaking, filtration, pre-extraction for antigen, etc. is optionally adopted in the measurement or assay process under specific conditions. The other assay conditions including the concentrations of specific reagents, buffers, etc., temperatures, incubation times, and the like can vary according to elements, such as the concentration of antigens in the sample, or the nature of samples to be measured. Any person ordinary skilled in the art can suitably select and determine optimal conditions effective for each assay while using the general experimentation and perform the selected measurement.

Various carriers on which antigens or antibodies can be immobilized are available in the art, and they can be arbitrarily and suitably selected in the present invention. For the immobilization, various carriers which can be used for antigen-antibody interactions are known. It goes without saying that any well-known carrier can be selected and used in the present invention. Preferred examples are inorganic materials including, for example, glass such as aminoalkylsilyl glass and other activated glass, porous glass, silica gel, silica-alumina, alumina, magnetized iron, magnetized alloy, etc.; organic polymer substances, such as polyethylene, polypropylene, polyvinyl chloride, polyvinylidene fluoride, polyvinyl, polyvinyl acetate, polycarbonate, polymethacrylate, polystyrene, styrene-butadiene copolymer, polyacrylamide, crosslinked polyacrylamide, styrene-methacrylate copolymer, polyglycidyl methacrylate, acrolein-ethylene glycol dimethacrylate copolymer, etc.; cross-linked albumin, collagen, gelatin, dextran, agarose, crosslinked agarose, natural and modified cellulose (for example, cellulose, microcrystalline cellulose, carboxymethylcellulose, cellulose acetate, etc.), crosslinked dextran, polyamides (for example, nylon, etc.), polyurethane, polyepoxy resin, etc.; products obtained by emulsion polymerization of such organic polymer substances; silicon gums, cells, erythrocytes, etc.; and such substances having a functional group introduced thereinto, as required, by using a silane coupling agent, etc..

Also included are solid materials (bodies) such as filter paper, beads, tubes, cuvettes, inner walls of test containers such as test tubes, titer plates, titer wells, microplates, glass cells, cells made of synthetic materials such as plastic resin cells, glass rods, rods made of synthetic materials, rods thickened or thinned at the end, rods provided with a round protrusion or a flat protrusion at the end, thin-plated rods, and surfaces thereof.

Antibodies can be coupled with these carriers. Preferably anti-galectin-9 antibodies (including antisera and purified antibodies) or monoclonal anti-galectin-9 antibodies, which are obtainable according to the present invention and react specifically with antigens, may be coupled to such a carrier as mentioned above. Coupling between the carrier and those partners associated with these antigen-antibody interactions can be carried out by techniques including physical method such as adsorption; a chemical method using a coupling agent, etc. or an activated reactant; a method using a chemically interactional coupling.

The label may include enzymes, enzyme substrates, enzyme inhibitors, prosthetic groups, coenzymes, enzyme precursors, apoenzymes, fluorescent substances, pigments, chemoluminescent compounds, luminescent substances, coloring substances, magnetic substances, metal particles such as gold colloids, radioactive substances and the like. The enzyme may include dehydrogenases, oxidoreductases such as reductases and oxidases; transferases that catalyze the transfer of functional groups such as amino, carboxyl, methyl, acyl, and phosphate groups; hydrolases that hydrolyze bonds such as ester, glycoside, ether, and peptide bonds; lyases; isomerases; ligases; and the like. Plural enzymes may be used in a conjugated form for detection (for example, enzymatic cycling may also be utilizable). Typical radioactive isotopes for the label include [³²P], [¹²⁵I], [¹³¹I], [³H], [¹⁴C], [³⁵S], etc. Typical enzymes for the label include peroxidases such as horseradish peroxidase; galactosidases such as E. coli beta-D-galactosidase; maleate dehydrogenases; glucose-6-phosphate dehydrogenases; glucose oxidases; gluocoamylases; acetylcholine esterases; catalases; alkaline phosphatases such as calf intestinal alkaline phosphatase and E. coli alkaline phosphatase, and the like. In the case where alkaline phosphatase is used, measurements can be done by monitoring or inspecting fluorescence, luminescence, etc., generated with a substrate such as umbelliferone derivatives including 4-methylumbellipheryl phosphate; phenol phosphate derivatives including nitrophenyl phosphate; enzymatic cycling systems utilizing NADP; luciferin derivatives; dioxetane derivatives; and the like. It is also possible to use a luciferin/luciferase system. When catalase is used, the reaction takes place with hydrogen peroxide to produce oxygen which can be detected with an electrode or the like. The electrode may be a glass electrode, an ionic electrode using an insoluble salt membrane, a liquid-membrane type electrode, a polymer membrane electrode and the like. The enzyme label may be replaced with a biotin label and an enzyme-labeled avidin (streptoavidin). Such use of a biotin-avidin system, use of a secondary antibody, for example, an antibody against an anti-galectin antibody, and other sensitivity-enhancing methods known in the art may be employed. For the label, a plurality of various kinds of labels or markers can be used. In this case, it is possible to perform plural measurements continuously or discontinuously and/or simultaneously or separately. According to the present invention, signal formation may be done using enzyme-reagent combinations, such as combinations of horseradish peroxidase or other peroxidases with a member selected from 4-hydroxyphenylacetic acid, o-phenylenediamine (OPD), tetramethylbenzidine (TMB), 5-aminosalicylic acid, 3,3-diaminobenzidine tetrahydrochloride (DAB), 3-amino-9-ethylcarbazole (AEC), tyramine, luminol, lucigenin-luciferin or derivatives thereof, Pholad luciferin, etc.; combinations of alkaline phosphatases with a member selected from lumigen PPD, (4-methyl)umbelliferyl phosphate, p-nitrophenol phosphate, phenol phosphate, bromochloroindolyl phosphate (BCIP), AMPAK™ (DAKO), AmPliQ™ (DAKO), etc.; combinations of beta-D-galactosidases or glucose-6-phosphate dehydrogenases with a member selected from 4-methylumbelliferyl-beta-D-galactoside or other umbelliferyl galactosides, o-nitrophenol-beta-D-galactoside, other nitrophenyl galactosides, etc.; combinations of glucose oxidases with ABTS, etc. The signal may be formed with those capable of enzymatically forming quinole compounds such as hydroquinone, hydroxybenzoquinone, and hydroxyanthraquinone, thiol compounds such as lipoic acid and glutathione, phenol derivatives or ferrocene derivatives.

The fluorescent substances and chemiluminescent compounds may include fluorescein isothiocyanate; Rhodamine derivatives such as Rhodamine B isothiocyanate and tetramethyl Rhodamine isothiocyanate (RITC), and tetramethylrhodamine isothiocyanate isomer R (TRITC); 7-amino-4-cumarin-3-acetic acid, dancyl chloride (5-(dimethylamino)-1-naphtalenesulfonyl chloride), dancyl fluoride, fluorescamine (4-phenylspiro[furan-2(3H),1'-(3'H)-isobenzofuran]-3,3'-dione), phycobiliprotein, acridinium salts; luminol compounds such as lumiferin, luciferase and aequorin; imidazoles, oxalic acid esters, rare earth chelate compounds, cumarin derivatives, etc. Although the generated signals, etc. (including luminescence, fluorescence, etc.) can be detected visually, they may be inspected or monitored with a known device, such as a fluorophotometer and a plate reader. For the detection of signals emitted by a radioactive isotope, etc., a known detector, such as a gamma counter and a scintillation counter, may be used.

The labelling can be accomplished by the reaction of a thiol group with a maleimide group, the reaction of a pyridyldisulfide group with a thiol group, the reaction of an amino group with an aldehyde group, etc. Additionally, it can be suitably selected from widely known methods, techniques which can be easily put into practice by an artisan skilled in the art, and any of modifications derived therefrom. The coupling agents used for producing the foregoing immunoconjugate or for coupling with carriers are also applicable and usable. The coupling agents include, for example, formaldehyde, glutaraldehyde, hexamethylene diisocyanate, hexamethylene diisothiocyanate, N,N'-polymethylene bisiodoacetamide, N,N'-ethylene bismaleimide, ethylene glycol bissuccinimidyl succinate, bisdiazobenzidine, 1-ethyl-3-(3-dimethylaminopropyl) carbodiimide, succinimidyl 3-(2-pyridyldithio)propionate (SPDP), N-succinimidyl 4-(N-maleimidometyl)cyclohexane-1-carboxylate (SMCC), N-sulfosuccinimidyl 4-(N-maleimidomethyl)-cyclohexane-1-carboxylate, N-succinimidyl (4-iodoacetyl)-aminobenzoate, N-succinimidyl 4-(1-maleimidophenyl)butyrate, N-(epsilon-maleimidocaproyloxy)succinimide (EMCS), iminothiolane, S-acetylmercaptosuccinic anhydride, methyl-3-(4'-dithiopyridyl)propionimidate, methyl-4-mercapto-butyrylimidate, methyl-3-mercaptopropionimidate, N-succinimidyl-S-acetylmercaptoacetate, etc.

According to the assay of the present invention, substances to be measured can be made to react sequentially with labeled antibody reagents (such as antisera, purified antibodies and monoclonal antibodies, labeled with enzymes or the like) and then with antibodies coupled on a carrier, or all the members can be reacted each other simultaneously. The order of adding reagents (members) may vary depending on the type of carrier system selected. In the case where sensitized beads such as sensitized plastic beads are used, the labeled antibody regents (such as labeled antisera, purified antibodies and monoclonal antibodies) are first put into a suitable test tube, together with a sample including substances to be measured, followed by addition of the sensitized plastic beads. Measurement can be then carried out.

For measurements (and/or detections) according to the present invention, the immunological measurement (immunoassay) is applied. For the measurement (assay), the solid phase carriers used may include various materials and shapes which can be selected from balls, microplates, sticks, microparticles, test tubes, and the like, made of polystyrene, polycarbonate, polypropylene, polyvinyl and other materials capable of adsorbing proteins such as antibodies.

The measurement can be carried out in a suitable buffer system so as to maintain an optimal pH (for example, between pH about 4 and about 9). The particularly preferred buffers may include acetate buffers, citrate buffers, phosphate buffers, Tris buffers, triethanolamine buffers, borate buffers, glycine buffers, carbonate buffers, Tris-HCl buffers, veronal buffers, etc. The buffers can be used optionally in a mixed form at any ratio. Preferably, the antigen-antibody interaction is carried out at a temperature between about 0 and 60°C.

The antibody (e.g., antiserum, purified antibody, monoclonal antibody, etc.) reagents labeled with enzymes or others, the immobilized antibody reagents (coupled to a carrier), and substances to be assayed can be incubated until equilibrium is reached. However, the reaction may be stopped after limited incubation wherein the solid phase is separated from the liquid phase at a time point well before the antigen-antibody interaction equilibrates, and the level of labels (such as enzymes) existing in either of the liquid and solid phases may be measured. Measurement operation can be performed with automated measuring instruments. A luminescence detector, a photo detector or the like may be used to measure or detect indication signals generated as a result of substrate conversion by the action of an enzyme.

In the antigen-antibody interaction, adequate means can be taken so as to stabilize regents to be used, samples to be assayed, and labels such as enzymes, respectively, and/or to stabilize antigen-antibody interactions per se. Further, proteins, stabilizers, surfactants, chelating agents or the like can be added to incubation solutions for eliminating non-specific reaction, reducing inhibitory influences acting thereon, and/or activating assay reaction. The chelating agent is more preferably ethylenediamine tetraacetate (EDTA). The blocking techniques for preventing non-specific binding reaction, which techniques are generally employed in the art or well-known among the persons skilled in the art, may be employed. The blocking can be achieved by treatments with mammal normal serum, serum proteins, albumin, hemoglobin, ovoalbumin (OVA), skim milk, fermented milk products, collagen, gelatin, or others. These methods or techniques can be used without any limitation so long as the use is for the purpose of preventing non-specific binding reaction. Furthermore, a suitable liquid may be selected and used from the above-mentioned buffers and salt solutions for washing the sample and solid phase. The liquid used may be admixed with a substance selected from the group consisting of Tween 20 (trade name), Tween 80 (trade name), NP-40 (trade name), Triton X100 (trade name), Briji (trade name), and other nonionic surfactants, CHAPS and other zwitterionic surfactants, cationic surfactants, and anionic surfactants.

The samples to be assayed according to the present invention may include various forms of solutions such as colloid solutions, non-fluid samples and the like. Preferably, the samples are biological samples including, for example, all organs and tissues, such as thymus, testis, intestine, kidney, brain, breast tissues, ovary, uterus, prostate gland, colon/rectum, stomach, lungs, bronchus, pancreas, and liver; malignant tumors of such organs and tissues, leukemic cells, blood, sera, plasma, articular fluid, cerebrospinal fluid, pancreatic juice, bile, saliva, amniotic fluid, urine, and other body fluids, cell culture medium, tissue culture medium, tissue homogenate, biopsy samples, tissues, cells, etc.

In applying various analytic and quantitative assays including those individual immunological assays (immunoassays) to the measurements (assays) of the present invention, it is unnecessary to set up therefor any special condition, operation, etc. Assay systems for the targets of the present invention or target substances having a substantially equivalent activity thereto may be constructed by adaptations of technical consideration ordinarily given by artisans in the art over general conditions and operations suitable for each of the methods.

Epitope mapping may also be carried out using the inventive anti-galectin-9 antibody, incuding especially monoclonal anti-galectin-9 Ab. Each galectin-9 and/or the respective galectin-9-related peptide fragments, etc. may be detected, measured or assayed with anti-galectin-9 antibodies that recognize the respective epitopes.

As aforementioned, the inventive cancer metastasis detecting agent containing anti-galectin-9 Ab in the form of polyclonal antibodies (including antisera) or monoclonal antibodies enables immunohistological analysis of galectin-9 levels in cancer cell samples extracted from cancerized tissues. That is, the metastatic potential of these cells can be detected or assessed by quantitative analysis of galectin-9 in examined cells.

As used herein, the cancer metastatic potential detecting agents comprise anti-galectin-9 Ab as an effective component. The phrase "comprise as an effective component" or "comprise an effective amount of" signifies that anti-galectin-9 Ab is contained at a concentration effective in the detection or assessment of cancer metastatic potential and/or cancer metastasis. The anti-galectin-9 Ab used may recognize any galectin-9 protein region, which is not restricted to an epitope.

For naturally-occurring galectin-9, galectin-9L, -9M, and -9S have been reported at present. Galectin-9S is considered to have its N-terminal domain linked to its C-terminal domain via the presumed link peptide region of SEQ ID NO: 4, galectin-9M is considered to have its N-terminal domain linked to its C-terminal domain via the presumed link peptide region of SEQ ID NO: 5, and galectin-9S is considered to have its N-terminal domain linked to its C-terminal domain via the presumed link peptide region of SEQ ID NO: 6. Galectin-9M differs from galectin-9L in lacking the amino acid residues of SEQ ID NO: 7 from the galectin-9L link peptide region. Galectin-9S differs from galectin-9M in lacking the amino acid residues of SEQ ID NO: 8 from the galectin-9M link peptide region, that is, galectin-9S differs from galectin-9L in lacking the amino acid residues of SEQ ID NO: 9 from the galectin-9L link peptide region. It may thus be construed that an object of this invention is also to provide anti-galectin-9 antibodies that can recognize such differences.

As used herein, the term "galectin-9" shall refer inclusively to the abovementioned galectin-9L, galectin-9M, galectin-9S, other naturally-occurring variants of the galectin-9 family, compounds obtained by incorporating artificial mutations (that is, deletions, additions, modifications, insertions with respect to one or more amino acid residues) into such galectin-9 peptides, and those containing a partial domain or partial peptide fragment thereof.

Representative anti-galectin-9 antibodies include antibodies that bind with all of galectin-9L, M, and S, antibodies that bind with both galectin-9L and M but do not react with galectin-9S, antibodies that bind with galectin-9L but do not react with either galectin-9M or S, antibodies that are respectively specific to galectin-9L, M, and S, antibodies that are specific to the C-terminal CRD of galectin-9 or a fragment peptide thereof, antibodies that are specific to the N-terminal CRD of galectin-9 or a fragment peptide thereof, antibodies that are specific to the respective link peptides or fragment peptides thereof, etc.

The measurement systems for galectin-9 include, for example, protein measurement systems, such as systems for immunostaining (METHODS, 24, 289-296 (2001); J. Immunol. Methods, 47(2), 129-144 (1981); ibid., 150 (1-2), 5-21, 23-32 & 151-158 (1992); Cancer J., 7(1), 24-31 (2001), etc.) and immunoelectron microscopy (Mol. Biotechnol., 7(2), 145-151 (1997); J. Electron Microsc. Tech., 19(1), 57-63 & 64-79 (1991); ibid., 19(3), 305-315 (1991), etc.), and expression gene measurement systems, such as in situ hybridization systems, used effectively for tissues; protein measurement systems, such as systems for EIA, RIA, FIA, LIA, and Western blotting (J. Electron Microsc. (Tokyo), 45(2), 119-127 (1996); Methods Biochem. Anal., 33, 1-58 (1988); Methods Enzymol., 271, 177-203 (1996); ibid., 305, 333-345 (2000); J. Immunol. Methods, 152(2), 227-236 (1992); ibid., 170(2), 177-184 (1994); ibid., 195(1-2), 149-152 (1996); Yoshiyuki Kuchino, et al. ed., "Idenshi-Tanpakushitsu, Jikken Sosa Burottingu-ho" (Genes and Proteins, Experimental Procedures, Blotting Methods), Soft Science Co., Ltd., Japan, Nov. 10, 1987, etc.), and expression gene measurement systems, such as systems for Northern blotting, dot blotting, RNase protection assay, RT-PCR (reverse transcription polymerase chain reaction), and real-time PCR (Clinical Chemistry, 46: 11, 1783-1743 (2000)), used effectively for tissue extracts; and protein measurement systems, such as systems for EIA, RIA, FIA, LIA, and Western blotting, used effectively for blood and body fluids, etc. The measurement systems for anti-galectin-9 antibodies include, for example, protein measurement systems, such as systems for EIA, RIA, FIA, LIA, and Western blotting, used advantageously for blood and body fluids, etc.

With regard to EIA measurement systems, for example competitive methods utilize solid-phase anti-galectin-9 Ab, a labeled antigen and a non-labeled antigen (the antigen may be galectin-9 or a fragment peptide thereof, etc.), while non-competitive methods, such as sandwich assays, do solid-phase anti-galectin-9 Ab, and labeled anti-galectin-9 Ab, as well as labeled or immobilized antibodies directed to anti-galectin-9 Ab without directly labeling or immobilizing anti-galectin-9 Ab. Sensitivity amplification or enhancement methods include, for example, combinations with non-enzyme-labeled primary Ab, including those using polymers, enzymes, primary Ab (adoptions of Envision reagents; Enhanced Polymer One-step Staining (EPOS)) and combinations with non-enzyme-labeled secondary Ab, including combinations of enzymes with anti-enzyme antibody conjujgates such as the PAP (peroxidase-antiperoxidase method), combinations of biotin-labeled secondary Ab with biotin-labeled enzyme-avidin complexes such as the SABC (avidin-biotinylated peroxidase complex method), combinations of biotin-labeled secondary Ab and biotin-labeled enzyme-streptavidin complexes such as the ABC (streptavidin-biotin complex method) and the LSAB (labeled streptavidin-biotin method), combinations of SABC with biotin-labeled tyramide and enzyme-labeled streptavidin such as the CSA (catalyzed signal amplification), methods in which a secondary antibody and an enzyme are labeled with a polymer, etc.

For details of those conventional technical methods, a variety of reviews, introductory texts, books, etc. may be referred to. They are, for example, Hiroshi Irie ed., "Radioimmunoassay", Kodansha, Japan, 1974; Hiroshi Irie ed., "Zoku-Radioimmunoassay" (Radioimmunoassay; Second Edition), Kodansha, Japan, 1979; Eiji Ishikawa et al. ed., "Koso Meneki Sokuteiho" (Enzyme Immunoassays), Igaku Shoin, Japan, 1978; Eiji Ishikawa et al. ed., "Koso Meneki Sokuteiho" (Enzyme Immunoassays) (2nd Ed.), Igaku Shoin, Japan, 1982; Eiji Ishikawa et al. ed., "Koso Meneki Sokuteiho" (Enzyme Immunoassays) (3rd Ed.), Igaku Shoin, Japan, 1987; H. V. Vunakis et al. (ed.), "Methods in Enzymology", Vol. 70 (Immunochemical Techniques, Part A), Academic Press, New York (1980); J. J. Langone et al. (ed.), "Methods in Enzymology", Vol. 73 (Immunochemical Techniques, Part B), Academic Press, New York (1981); J. J. Langone et al. (ed.), "Methods in Enzymology", Vol. 74 (Immunochemical Techniques, Part C), Academic Press, New York (1981); J. J. Langone et al. (ed.), "Methods in Enzymology", Vol. 84 (Immunochemical Techniques, Part D: Selected Immunoassays), Academic Press, New York (1982); J. J. Langone et al. (ed.), "Methods in Enzymology", Vol. 92 (Immunochemical Techniques, Part E: Monoclonal Antibodies and General Immunoassay Methods), Academic Press, New York (1983); J. J. Langone et al. (ed.), "Methods in Enzymology", Vol. 121 (Immunochemical Techniques, Part I: Hybridoma Technology and Monoclonal Antibodies), Academic Press, New York (1986); J. J. Langone et al. (ed.), "Methods in Enzymology", Vol. 178 (Antibodies, Antigens, and Molecular Mimicry), Academic Press, New York (1989); M. Wilchek et al. (ed.), "Methods in Enzymology", Vol. 184 (Avidin-Biotin Technology), Academic Press, New York (1990); J. J. Langone et al. (ed.), "Methods in Enzymology", Vol. 203 (Molecular Design and Modeling: Concepts and Applications, Part B: Anibodies and Antigens, Nucleic Acids, Polysaccharides, and Drugs), Academic Press, New York (1991); etc. and references quoted in the above documents, the disclosures of which are incorporated herein by reference.

In another mode, the present invention provides cancer metastatic potential-detecting agents, cancer metastatic potential detection methods (assays), and cancer metastatic pote ntial-detecting systems for such methods, which comprise an effective amount of a nucleic acid (or oligo- or poly-nucleotide) that hybridizes with a nucleic acid coding for galectin-9 or a constituent domain thereof. The nucleic acid to be hybridized may include, for example, probes, primers, etc. Any probe that hybridizes with a galectin-9 gene or a product thereof can be applied without limitation as long as it suits the purpose. The nucleic acids may be obtained according to the aforementined "gene recombination techniques". For example, the mucleic acid may be obtained readily by designing and synthesizing a plurality of primers, relying on known information on the nucleotide base sequence of galectin-9, and carrying out PCR (polymerase chain reaction), etc. The primers may be prepared by methods known in the art. Representatively, the primers may be synthesized by a phosphodiester method, phosphotriester method, phosphoramidite method, etc.. The primers may be synthesized, for example, with an automated DNA synthesizer, such as Model 381A DNA Synthesizer (Applied Biosystems), etc. PCR may be carried out using a cDNA library, sense primer, and anti-sense primer to amplify the cDNA. The nucleic acid thus obtained may be used as a specific hybridization probe. The probe, etc. may be labeled by a radioactive isotope using a commercially available labeling kit, such as the Random Prime DNA Labeling Kit (Boehringer Manheim), etc. For example, a random priming kit (Pharmacia LKB, Uppsala), etc. may be used to label the probe DNA with [α -³²P]dCTP (Amersham), etc. and thus provide a probe with radioactivity. The probe label used herein include those known in the art and suitably selected from those disclosed for antibodies.

The hybridization is achieved by transferring a sample containing a target DNA, etc. onto membranes such as nylon filters, as required, optionally followed by denaturation, fixation, washing, etc., and then reacting the transfers on the membrane, with labeled DNA probe fragments which are, as required, optionally denatured in a hybridization buffer. The hybridization operations can be ordinarily conducted at about 35 to about 80°C, more preferably about 50 to about 65°C, for about 15 min to about 36 hours, more preferably about 1 to about 24 hours, but optimal hybridization conditions may be suitably selected. For example, the hybridization is carried out at about 55°C for about 18 hours. The hybridization buffers can be selected from those customarily used in the art. Examples of the hybridization buffers are Rapid hybridization buffer (Amersham), etc. The denaturation of membranes with transfers includes techniques using an alkali denaturing solution. It is preferable to treat the membrane with a neutralizing solution and a buffer solution after the denaturation. The membrane fixation is usually achieved by baking at about 40° to about 100°C, more preferably about 70° to about 90°C, for about 15 min to about 24 hours, more preferably about 1 to about 4 hours, but desired fixation conditions may be suitably selected. For example, the fixation is carried out by baking a filter at about 80°C for about 2 hours. The washing of membranes with transfers can be performed with washing solutions customarily used in the art, such as 50mM Tris-HC1 buffer, pH8.0, containing 1M NaCl, 1mM EDTA and 0.1% sodium dodecyl sulfate (SDS). The membranes such as nylon filters can be selected from those customarily used in the art. Examples of the membrane are nylon filters (Hybond-N, Amersham), etc.

The alkaline denaturing solution, neutralizing solution and buffer solution can be selected from those conventionally used in the art. The alkaline denaturing solution may include, for example, solutions containing 0.5M NaOH and 1.5M NaCl, etc. The neutralizing solution may include, for example, 0.5M Tris-HCl buffers (pH8.0) containing 1.5M NaCl, etc. The buffer solution may include, for example, 2 x SSPE (0.36M NaCl, 20mM NaH₂PO₄ and 2mM EDTA), etc. As required, prior to hybridization, it is desired to optionally prehybridize membranes containing transferred DNA, etc., to prevent non-specific hybridization. For the prehybridization, the sample is dipped, for example, in a solution for prehybridization (50% formamide, 5 x Denhardt's solution (0.2% bovine serum albumin and 0.2% polyvinylpyrrolidone), 5 x SSPE, 0.1% SDS, and 100 µ g/ml thermally denatured salmon sperm DNA), etc., and reacted at about 35 to 50°C, preferably about 42°C, for about 4 to 24 hours, preferably about 6 to 8 hours. These conditions can be determined by those of skill in the art with suitably repeated experiments and preferred conditions would be selected. Labeled probe DNA fragments used in hybridization can be denatured, for example, under heating conditions at about 70 to 100°C, preferably about 100°C, for about 1 to 60 minutes, preferably about 5 minutes, etc. The hybridization is carried out by well known techniques per se in the art or according to methods analogous thereto. As used herein, the stringent conditions refer to, for example, those equivalent to hybridization in about 15 to 50 mM, preferably about 19 to 40 mM, and more preferably about 19 to 20 mM, with regard to Na ion concentration, at about 35 to 85°C, preferably about 50 to 70°C, and more preferably about 60 to 65°C with regard to temperature.

After the hybridization is completed, the filters are washed extensively to remove labeled probes other than the labeled probe DNA fragments which specifically hybridize. The filter washing process may be performed by a method suitably selected from techniques used in the art. For example, the washing is carried out in 0.5 x SSC solution (x SSC= 0.15M NaCl, 15mM citric acid) containing 0.1% SDS. The hybridized spots can be detected representatively by autoradiography, but the detection may be performed by a method suitably selected from techniques used in the art.

Cancer metastatic potential (and/or cancer metastatic properties) can be detected or assessed by detecting and/or measuring and/or assaying galectin-9 expression genes (including DNA such as cDNA and RNA such as mRNA) according to the aforementioned "gene recombination techniques". The metastatic potential (and/or metastatic property) of cancers may be detected or assessed with detection and/or measur ement methods and/or assays for the expression of specific genes, known per se in the art. Such methods and/or assays may include, for example, in situ hybridization, Northern blotting, dot blotting, RNase protection assay, RT-PCR, real-time PCR (Journal of Molecular Endocrinology, 25, 169-193 (2000) and reference documents quoted therein), DNA array analysis (Mark Sheen ed., "Microarray Biochip Technology", Eaton Publishing (March, 2000)), etc. Not only galectin-9 expression gene measurement systems utilizing such techniques but also reagents, methods (and/or assays), processes, etc. used in such systems are all included in this inventive cancer metastatic potential detection (or assessment) agent, cancer metastatic potential detection and/or measurement method (and/or assay), and system for the method (and/or assay). The in situ hybridization may include, for example, non-RI in situ hybridization, direct and indirect methods. The direct method includes techniques utilizing, for example, a detectable molecule (reporter) directly bound to a nucleic acid probe to enhance or amplify a signal, while the indirect method includes techniques utilizing, for example, an antibody against the reporter molecule for the signal enhancement or amplification. The oligonucleotide in the nucleic acid probe may have an incorporated functional group (for example, a primary aliphatic amino group, SH group, etc.) to which a hapten, fluorescent pigment, enzyme, etc. may be bound. Representatives of the nucleic acid probe labels include digoxygenin (DIG), biotin, fluorescein, etc. The nucleic acid probe label used may be a label suitably selected from those described herein for antibodies. Multiple labeling is also utilizable and further a labeled antibody may be adopted. Nucleic probe labeling methods may be suitably selected from those known in the art. Examples of such labeling methods include random priming methods, nick translation methods, PCR amplifications of DNA, labeling/tailing methods, in vitro transcription methods, etc. Treated samples can be observed with methods suitably selected form those known in the art. Examples of such methods used are those utilizing a dark-field microscope, a phase contrast microscope, a reflection contrast microscope, a fluorescence microscope, a digital imaging microscope, an electron microscope, etc. The observation may also be made by flow cytometry, etc. In accordance with the present invention, galectin-9 and a galectin-9 expression gene can be used as cancer metastasis markers or tumor markers, thereby leading to the preparation and/or construction of various forms of cancer metastatic potential detection agents or tumor detection and/or measurement agents, cancer metastatic potential detection methods or tumor detection and/or measurement methods (assays), and cancer metastatic potential detection or tumor detection and/or measurement reagent sets or systems, which are not only helpful but also excellently effective for the diagnosis, prophylaxis, and/or therapy of cancers. Furthermore, these may be arranged as cancer metastatic potential detecting agents or tumor detection and/or measurement agents, cancer metastatic potential detection methods or tumor detection and/or measurement methods (assays), and cancer metastatic potential detection or tumor detection and/or measurement reagent sets or systems for the stages after the therapy, i.e., post-treatments against cancer, thereby providing excellent functions, actions, and effects for prognostic purposes.

As used herein, "marker" may cover species that enable recognition or identification of "metastatic potential" or "tumor", and tools that function as measures for the degree or level of "metastatic potential" or "metastatic property" or for the malignancy of a "tumor". It may be considered that the above-described functions are indicated by the existence/non-existence and/or quantitative differences of the marker.

For terms (words) and/or abbreviations used in the specification and in the drawings, they must conform with an "IUPAC-IUB Commission on Biochemical Nomenclature" or are based on the meanings of the terms which are commonly used in the art.

### Examples

Described below are examples of the present invention which are provided only for illustrative purposes, and not to limit the scope of the present invention. In light of the present disclosure, numerous embodiments within scope of the claims will be apparent to those of ordinary skill in the art. All the examples were or can be practiced using standard techniques well or conventionally known to those of ordinary skill in the art unless otherwise specified.

Unless specifically indicated otherwise, specific procedures, operations, treatment conditions, etc. in examples as described herein below are conducted or selected according to the methods or techniques disclosed in, for DNA cloning, J. Sambrook, E. F. Fritsch & T. Maniatis, "Molecular Cloning", 2nd ed., Cold Spring Harbor Laboratory, Cold Spring Harbor, N. Y. (1989) and D. M. Glover et al. ed., "DNA Cloning", 2nd ed., Vol. 1 to 4, (The Practical Approach Series), IRL Press, Oxford University Press (1995); and, particularly for applications of PCR, H. A. Erlich ed., PCR Technology, Stockton Press, 1989; D. M. Glover et al. ed., "DNA Cloning", 2nd ed., Vol. 1, (The Practical Approach Series), IRL Press, Oxford University Press (1995) and M. A. Innis et al. ed., "PCR Protocols", Academic Press, New York (1990). When commercially available reagents and kits are used, protocols, chemicals, etc. attached thereto are employed herein.

### Example 1

### Preparation of anti-galectin-9 antibody-containing antisera and purification of anti-galectin-9 antibody

Among the two carbohydrate recognition domains (CRD's) of human galectin-9, the C-terminal CRD (protein having a sequence corresponding to amino acid residues 192 to 355 of galectin-9L, SEQ ID NO: 1) was selected and prepared as a sensitizing antigen. First, the base sequence coding for the galectin-9L C-terminal CRD was incorporated into a vector (pGEX-4T-2, Amersham Pharmacia Biotech Inc.) to construct an expression vector for the production of a fused protein comprised of glutathione S-transferase (GST) and the C-terminal CRD. Escherichia coli (BL21, Amersham Pharmacia Biotech Inc.) was transformed with this expression vector to express intracellularly the aforementioned fused protein. The Escherichia coli cells were then disrupted and the resultant supernatant was fractionated on a glutathione Sepharose column (Amersham Pharmacia Biotech Inc.). The fused protein contained in the supernatant was thus purified.

Next, thrombin (Amersham Pharmacia Biotech Inc.) was reacted with a solution containing the purified fused protein to thereby cleave the C-terminal CRD and GST bond. The C-terminal CRD was purified by affinity chromatography on a lactose column (Honen Corporation, Japan).

The C-terminal CRD (200 µ g) was mixed in 1ml of Freund's complete adjuvant (Difco Inc.) to form a sensitizing antigen-containing solution. This solution was subcutaneously injected (5 times) into a rabbit (3 to 4kg weight, Japanese house rabbits, Japan SLC Inc.) at an amount of 1 ml every 2 weeks.

One week later after the 5th subcutaneous injection, blood was collected from the immunized rabbit and a serum was prepared. Antibodies contained in the resultant serum were verified to recognize galectin-9 specifically and this serum was consequently used as an antiserum containing anti-galectin-9 Ab.

Meanwhile for comparison, the entire molecule of galectin-1 was used as an antigen to prepare an antiserum containing anti-galectin-1 Ab in the same procedures as described above. Similarly, the the entire molecule of galectin-3 was used as an antigen to prepare an antiserum containing anti-galectin-3 Ab. The anti-galectin-1 Ab and the anti-galectin-3 Ab specifically recognized galectin-1 and galectin-3, respectively. Mutual cross reactions were not seen among the anti-galectin-9 Ab, anti-galectin-1 Ab, and anti-galectin-3 Ab.

Purified anti-galectin-9 Ab was prepared by affinity chromatography on a column in which purified galectin-9 C-terminal CRD was fixed to Affigel (Bio-Rad Corp.). Purified galectin-9 C-terminal CRD (10mg) was mixed with 10ml of Affigel-10 (Bio-Rad Corp.), and allowed to stand at 4°C overnight. The resultant Affigel was washed extensively with 20mM phosphate-bufferred physiological saline solution (PBS), and unreacted functional groups on the Affigel were blocked with PBS containing 100mM monoethanolamine overnight. In the final step, the Affigel was washed with PBS again and a galectin-9 C-terminal CRD-affixed column was prepared. Anti-galectin-9 Ab-containing rabbit serum (10 ml) was applied to this affixed column. After washing extensively with PBS, adsorbed anti-galectin-9 Ab was eluted with 20mM glycine hydrochloride buffer solution (pH 4.0). The eluted anti-galectin-9 Ab solution was immediately neutralized with 200mM Tris-HCl buffer, and after dialyzing overnight with PBS, cryopreserved at -80°C.

### Example 2

### Metastatic potential detection using melanoma cells

The metastatic potential of melanoma cells was detected with anti-galectin-9 Ab-containing antiserum as prepared in Example 1.

For melanoma cells used in the present Example 2, excised samples selected from primary tumor tissues (primary foci): 75 examples, metastatic cells (metastatic foci): 33 examples, and pigmented nevi: 28 examples were used as shown in Table 1.

**Table 1**

| | Primary Tumor | Metastatic | Pigmented Nevus |
|---|---|---|---|
| Number of samples | 75 | 33 | 28 |
| Male/female | 33/42 | 13/9 | 7/21 |
| Average age | 64.9 | 64.5 | 21.2 |
| Age range | 21-90 | 22-90 | 8-55 |
| Stage | | | |
| 0/I/II/III/IV | 4/22/20/25/4 | | |
| Tissue type | | | |
| melanoma in situ | 4 | | Junction type 2 |
| acral lentiginous melanoma | 37 | | Compound type 1 |
| superficial spreading melanoma | 35 | | Intradermal type 25 |
| nodular melanorma | 7 | | |
| Mucous melanoma | 5 | | |
| Lentigo maligna melanoma | 7 | | |
| Average thickness (mm) | 3.2+/-2.6 | | |
| (Range) | (0.1-10) | | |

The expression of galectin-9 in these melanoma cells was assayed with Example 1 anti-galectin-9 Ab-containing antiserum. A sample wherein the expression of galectin-9 has been detected with anti-galectin-9 Ab contained in the antiserum is deemed to be positive, a sample wherein the galectin-9 expression has not been observed is deemed to be negative, and a sample wherein only a slight galectin-9 expression has been detected is deemed to be weakly positive. The results are shown in Table 2.

**Table 2**

| | Galectin-9 | |
|---|---|---|
| | Strongly positive (%) | Weakly positive and negative (%) |
| Pigmented nevus | 20 (71) | 8 (29) |
| Primary malignant melanoma | 29 29 (39) | 46 (61) |
| Metastatic malignant melanoma | 10 (30) | 23 (70) |

As apparent from Table 2, positive cells were significantly more than negative cells for benign pigmented nevi. For malignant pigmented nevi, significantly less positive results were obtained than negative results. Also, though there were comparatively many negative results for primary tumor and metastatic foci, a statistically significant difference was not obtained.

Next, the primary foci (75 examples) were examined for the relationship between their tumor thickness and galectin-9 expression level. Examples wherein the tumor thickness was 1.5 mm or less were grouped as "A group", those wherein greater than 1.5 mm but less than 4 mm as "B group", and those wherein 4 mm or greater as "C group", respectively. Cases wherein the melanoma cells were positive at 80% or more were deemed to be of a strongly positive group and others were deemed to be of a negative group.

| | | |
|---|---|---|
| A group: | Strongly positive group 2 examples, | Negative group 25 examples |
| B group: | Strongly positive group 10 examples, | Negative group 12 examples |
| C group: | Strongly positive group 17 examples, | Negative group 9 examples |

From these results, it can be understood that, in cancerization processes, galectin-9 first disappears and is thereafter expressed in larger amounts in accompaniment with the growth of melanoma cells (that is, with an increase in tumor thickness).

Of these 75 primary focus examples, lymphatic metastasis was observed for 15 examples at the initial diagnosis. For these 15 examples, the measurement results of the relationship between the galectin-9 expression level and tumor thickness were as follows:

| | | |
|---|---|---|
| A group : | Strongly positive group 0 examples, | Negative group 1 example |
| B group : | Strongly positive group 1 example, | Negative group 1 example |
| C group : | Strongly positive group 3 examples, | Negative group 9 examples |

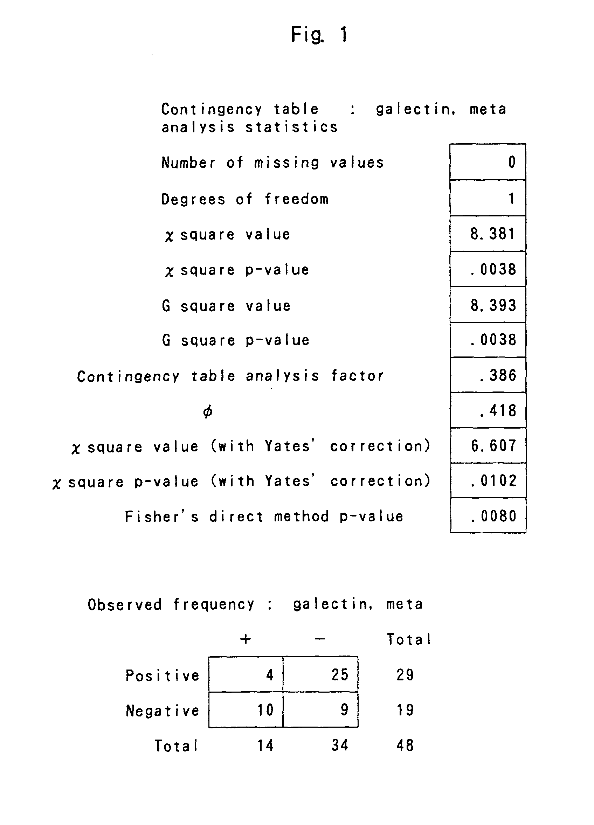

Among the 75 primary focus examples based on these results, were statistically studied 48 examples (B group and C group) which had a tumor thickness of greater than 1.5 mm. The conditions and results for the statistical study are shown in Fig. 1. As can be understood from Fig. 1, a correlation between galectin-9 expression and lymphatic metastasis can be seen for the primary focus group with a tumor thickness of greater than 1.5 mm. That is, it can be understood that the negative group is significantly greater than the strongly positive group for the 14 examples which belong to the primary focus group with a tumor thickness of greater than 1.5 mm and gave rise to lymphatic metastasis.

Furthermore, postoperative recurrence was seen with 12 primary focus examples among the 75 examples. For these 12 examples, the measurement results of the relationship between galectin-9 expression level and tumor thickness are as follows:

| | | |
|---|---|---|
| A group : | Strongly positive group 0 examples, | Negative group 1 example |
| B group : | Strongly positive group 0 examples, | Negative group 0 examples |
| C group : | Strongly positive group 2 examples, | Negative group 9 examples |

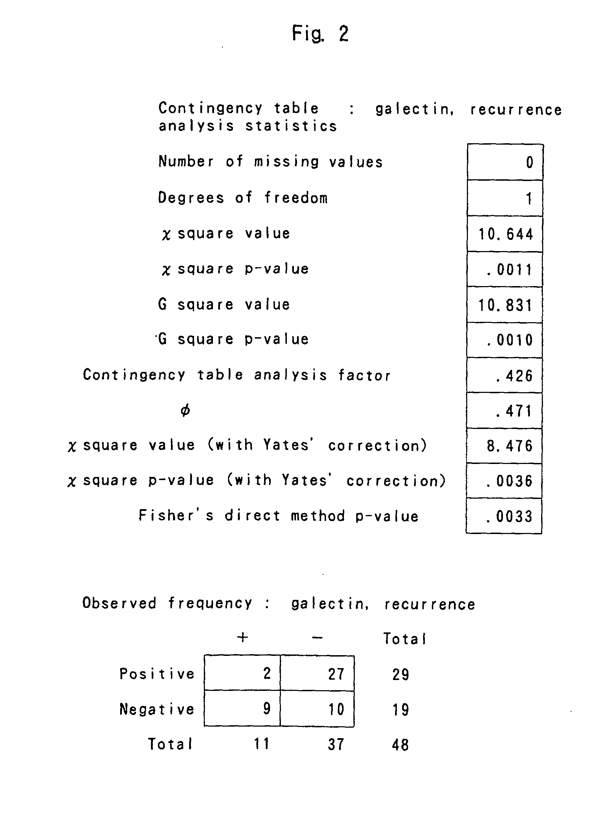

Among the 75 primary focus examples based on these results, a statistical study was carried out using 48 examples (B and C groups) which had a tumor thickness of greater than 1.5 mm. The conditions and results for the statistical study are shown in Fig. 2. As can be understood from Fig. 2, a correlation between galectin-9 expression and post-operative recurrence can be seen for the primary focus group with a tumor thickness of greater than 1.5 mm. That is, it can be understood that the negative group is significantly greater than the strongly positive group for the 11 examples which belong to the primary focus group with a tumor thickness of greater than 1.5 mm, and bear postoperative recurrence.

Furthermore, death occurred in 13 primary focus examples among the 75 examples. The measurement results of the relationship between galectin-9 expression level and tumor thickness for these 13 examples are as follows:

| | | |
|---|---|---|
| A group : | Strongly positive group 0 examples, | Negative group 1 example |
| B group : | Strongly positive group 0 examples, | Negative group 1 example |
| C group : | Strongly positive group 3 examples, | Negative group 8 examples |

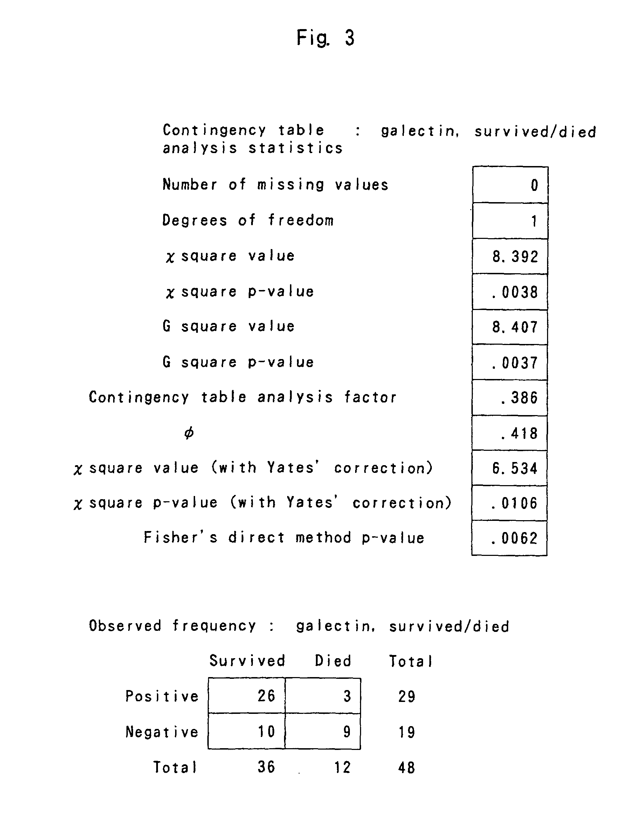

Among the 75 primary focus examples based on these results, a statistical study was carried out using 48 examples (B and C groups) which had a tumor thickness of greater than 1.5 mm. The conditions and results for the statistical study are shown in Fig. 3. As can be understood from Fig. 3, a correlation between galectin-9 expression and death can be seen for the primary focus group with a tumor thickness of greater than 1.5 mm. That is, it can be understood that the negative group is significantly greater than the strongly positive group for the 12 examples which belong to the primary focus group with tumor thickness of greater than 1.5 mm and have died.

From these results (Figs. 1 through 3), it can be understood that galectin-9 expression-positive melanoma cells are low in metastatic potential. It has thus been clearly shown that an anti-galectin-9 Ab-containing antiserum enables detection (and/or assessment) of the metastatic potential of cancer cells and can be used as a cancer cell metastatic potential-detecting agent.

### Example 3

### Metastatic potential detection using mammary cancer cells

The metastatic potential of mammary cancer cells (breast cancer cells) was detected with anti-galectin Ab-containing antiserum as prepared in Example 1.

Mammary cancer cells obtained from 67 mammary cancer patients were used in this example. These mammary cancer cells were pathohistologically classified into papillotubular carcinomas: 20 examples, solid-tubular carcinomas: 25 examples, and scirrhous carcinomas: 22 examples. The mammary cancer cells obtained were checked for galectin-9 expression with the anti-galectin Ab-containing antiserum obtained in Example 1. The results are shown in Table 3.

**Table 3**

| Galectin-9 | Positive | Negative | % positive |
|---|---|---|---|
| Papillotubular carcinoma | 13 | 7 | 65% |
| Solid-tubular carcinoma | 11 | 14 | 44% |
| Scirrhous carcinoma | 5 | 16 | 27% |
| Total | 45% | 55% | 100% |

From Table 3, it can be understood that the highest galectin-9 positive percentage value is indicated for papillotubular carcinoma cases, which are considered to be of good prognosis.

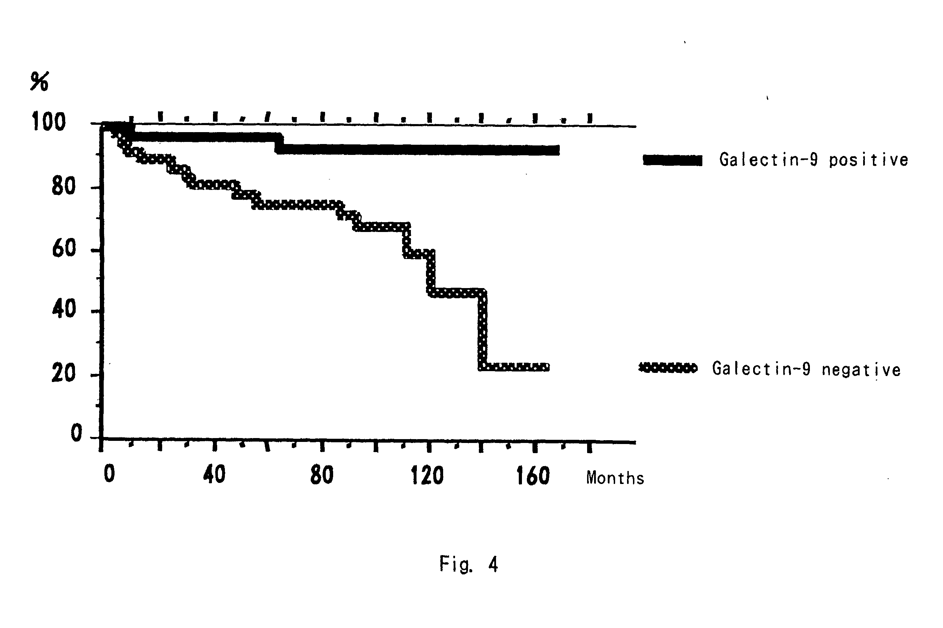

Also, of the 16 mammary cancer cell examples, 14 examples were galectin-9 negative among the 32 examples which lead to distant metastasis. The non-metastatic cumulative survival rates were measured for the galectin-9 positive and negative groups. The measurement results are shown in Fig. 4. As can be understood from Fig. 4, a significant difference in non-metastatic cumulative survival rate was seen between the galectin-9-positive and -negative groups. Furthermore, the correlation between lymphatic metastasis, estrogen receptor expression rate, and distant metastasis was examined for the galectin-9-positive and -negative groups. The results are shown in Table 4. In Table 4, the lymphatic metastasis is indicated by "n(+)", the estrogen receptor expression rate is indicated as "ER," and the distant metastasis is indicated by "meta."

**Table 4**

| | n(+) | ER | meta |
|---|---|---|---|
| Positive | 43% | 50% | 6% |
| Negative | 41% | 76% | 38% |
| Significant difference | None | None | 0.003 |

As can be understood from Table 4, there was neither significant difference nor conceivable correlation among lymphatic metastases, estrogen receptor expression rates, and galectin-9 expressions. However, a significant difference and a correlation could be seen between distant metastasis and galectin-9 expression.

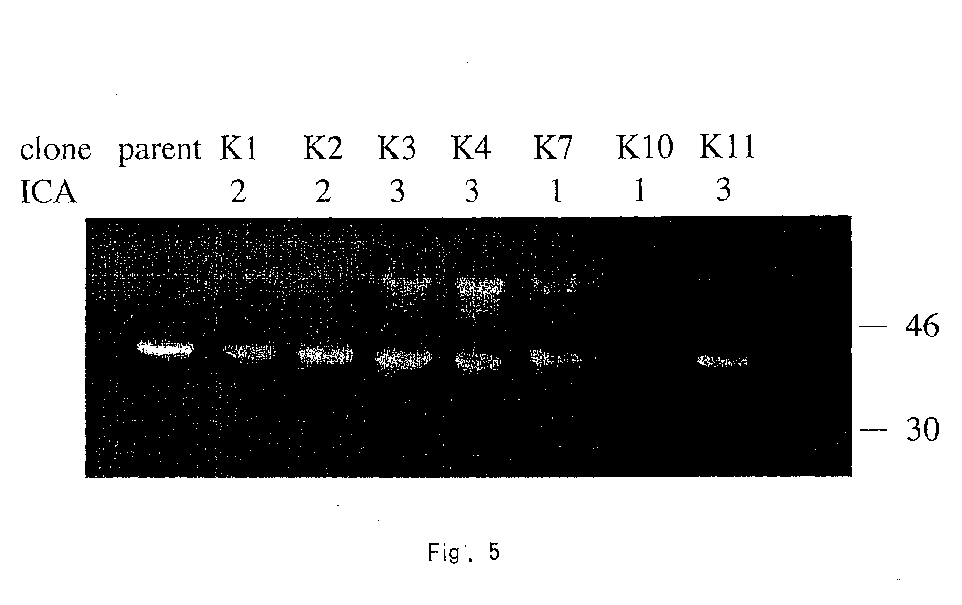

Meanwhile, screening for highly galectin-9-expressing and poorly galectin-9 expressing lines was carried out according to subcloning techniques known in the art. As a result, 5 highly galectin-9 expressing cell lines and 2 poorly galectin-9 expressing cell lines were obtained as shown in Fig. 5. Fig. 5 is an electrophoretogram, illustrating the galectin-9 expression results obtained by Western blotting. In Fig. 5, the "parent" cell lane is a control that expresses galectin-9.

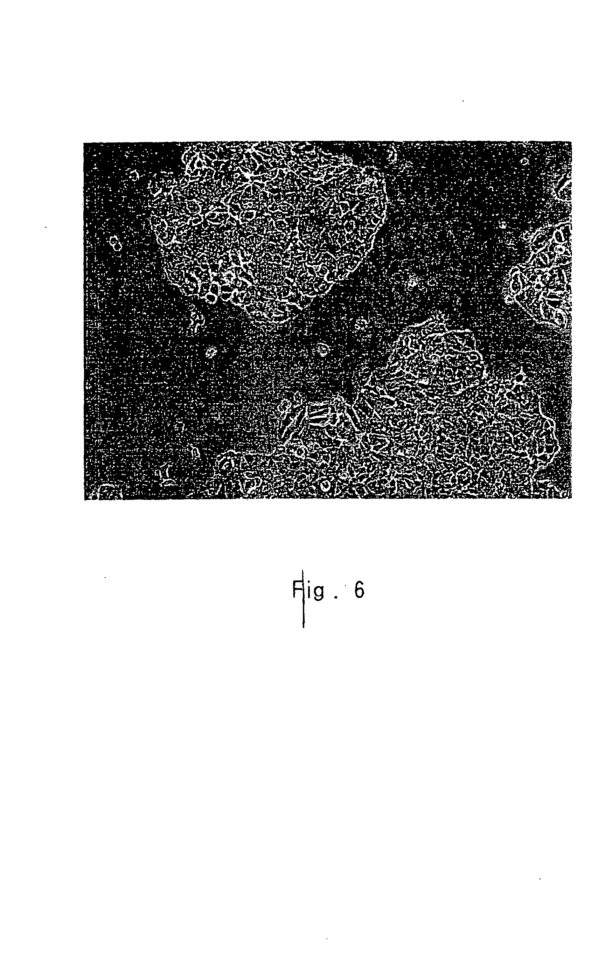

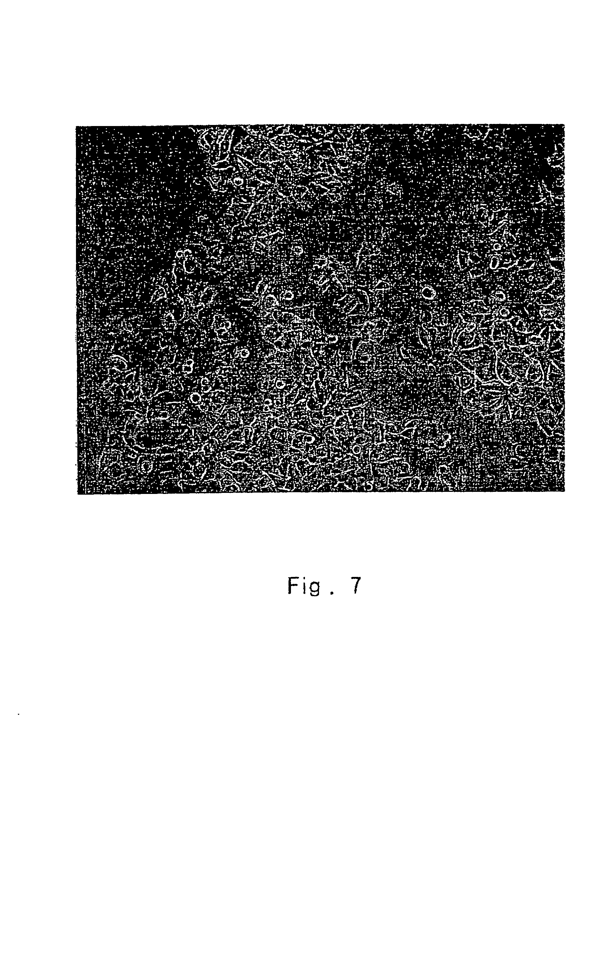

Culturing of a highly galectin-9 expressing cell line (the lane indicated as K4 in Fig. 5) resulted in a cell mass in which a plurality of cells were adhered together, as shown in Fig. 6. In contrast, culturing of a poorly galectin-9 expressing cell line (the lane indicated as K10 in Fig. 5) resulted in a plurality of cells growing in a dispersed manner, as shown in Fig. 7.

These results indicate that galectin-9 strongly maintains intercellular adhesion of mammary cancer cells and restrains the separation of mammary cells from a cell mass (tumor mass). In other words, it has been indicated that poorly galectin-9 expressing cell lines separate readily from a cell mass and consequently causes cancer metastasis. It has thus become apparent that the metastatic potential of targeted mammary cancer cells can be detected and/or assessed by measuring (and/or assaying) the expression of galectin-9 in mammary cancer cells with an anti-galectin-9 Ab-containing antiserum.

### Example 4

### Galectin-9 detection by immunohistological staining

The following processes were performed on paraffin embedded section samples to detect galectin-9 by immunohistological staining and to examine their cancer metastatic potential:

The deparaffinization process was carried out 3 times with xylene (10 min each) and once with 100% alcohol, 90% alcohol, and 75% alcohol sequentially (2 min each). Microwave (MW) treatment was performed by dispensing 10 mM citric acid buffer (pH 6.0) into two heat-resistant staining jars followed by boiling with MW irradiation. One of the staining jars was used for a section sample and the other was used for additional buffer. Each section sample was placed in the boiled buffer and subjected to MW irradiation (5 min x 2 times, total 10 min, 500W microwave oven). In order to replenish the evaporated buffer, heating was performed while adding boiled buffer every 5 min. The treated sections were allowed to stand at room temperature for 20 min and cooled gradually.

In order to block endogenic peroxidases, 0.3% peroxide-methanol was prepared when necessary and the section sample was dipped thereinto for 30 min. The section was then washed 4 times with PBS (phosphate buffered saline) (the section was dipped for 10 min for the 4th wash). After washing, the moisture in the peripheral tissue was wiped off with filter paper and the section was placed in a wet box. An aliquot (6 drops) of PBS (containing 0.2% sodium azide) containing 5% BSA (bovine serum albumin) was dropped onto the section which was then placed in a wet box at room temperature for 1 hr for blocking.

Onto the BSA over the tissue sample (without washing) were applied 6 drops of a 2% BSA-PBS solution containing a primary antibody (anti-galectin-9 Ab, 10µ g/ml), and the sample was then allowed to stand in a wet box at room temperature overnight to react with the primary antibody. The sample was then washed 4 times with PBS (the sample was dipped for 10 min for the 4th wash). After washing, the moisture in the peripheral tissue was wiped off with filter paper and the sample was placed in a wet box. An aliquot (6 drops) of second antibody, horseradish peroxidase (HRP)-labeled anti-rabbit Ab (DACO ENVISION, K4001), was applied onto the tissue sample. After then placing the tissue sample in a wet box at room temperature for 1 hr, the tissue sample was washed 4 times with PBS. The visualization procedure was then carried out with a DAB (3,3'-diaminobenzidine tetrahydrochloride) reagent (DAB + PBS + 30% hydrogen peroxide). The coloring process was observed and stopped immediately prior to coloration of the control and the coloring reaction was stopped by washing with water 8 times.

Nuclear staining was performed with Mayer's hematoxylin. Dehydration, penetration, and sealing were performed with 75% alcohol, 90% alcohol, and 100% alcohol (2 min each), respectively, and with xylene 3 times (3 min each) followed by sealing.

Dilutions (5, 10, and 20µ g/ml) of anti-galectin-9 Ab and normal rabbit IgG (control antibody) were prepared, respectively. The sections of 2 mammary cancer examples (cancer tissue of papillotubular carcinoma (relatively good prognosis) and scirrhous carcinoma (relatively bad prognosis)) were stained.

When the 5µ g/ml solutions were used, the staining due to the control antibody was weak and the staining by anti-galectin-9 Ab was judged to be weak as well. When the 10µ g/ml solutions were used, the difference between the staining due to the control antibody and that by anti-galectin-9 Ab was adequately clear though the staining due to the control antibody was not zero, and specific staining was achievable. With cases where the 20µ g/ml solutions were used, the staining due to anti-galectin-9 Ab was extremely intense though the staining due to the control antibody was also intense.

It was thus found that the use of anti-galectin-9 Ab enables the detection of galectin-9 expression tissues by immunohistologically staining cancer tissues and further the evaluation or assessment of galectin-9 expression levels (quantitative evaluation between large and small amounts of galectin-9).

### Example 5

### Preparation of monoclonal antibodies

### (a) Immunogens

As the antigen to be used for immunization, recombinant galectin-9L or -9M or a synthetic peptide designed based on an amino acid sequence selected from SEQ ID NOs: 1, 4, 5, 6, 7, 8, and 9 on the Sequence Listing may be used. Furthermore, as the antigen to be used for immunization, a recombinant protein may be used, which is obtained by ligating obtained cDNA to an animal cell expression vector followed by expression of this vector in a CHO or COS cell, etc. Such antigen proteins may be purified by gel filtration, ion exchange, affinity chromatography or other various chromatography techniques.

The purified immunization antigen may be subjected to immunization according to a generally used method to raise antibody-producing cells, which are employed for cell fusion to produce antibody-producing hybridoma cells. Furthermore, cloning may be performed based on reactivity against the purified immunization antigen wherein monoclonal antibody-producing hybridoma cell lines can be established.

Also, as the immunogen for obtaining monoclonal Ab specific to galectin-9L, -9M, and/or -9S, a synthetic haptenized peptide, having an amino acid sequence that is characteristic to each form of galectin-9, may be used. For example, it is especially preferable that peptides contain at least three or more continuous amino acid resisues, and preferably five or more amino acid resisues selected from the sequence from Met¹ to Thr¹⁶⁷ of SEQ ID NO: 2 in the Sequence Listing, the sequence from Ile¹⁹² to Thr³⁵⁵ of SEQ ID NO: 1 in the Sequence Listing, the sequence from Ile¹⁶⁰ to Thr³²³ of SEQ ID NO: 2 in the Sequence Listing, the presumed link peptide domains:

Asn-Pro-Arg-Thr-Val-Pro-Val-Gln-Pro-Ala-Phe-Ser-Thr-Val-Pro-Phe-
Ser-Gln-Pro-Val-Cys-Phe-Pro-Pro-Arg-Pro-Arg-Gly-Arg-Arg-Gln-Lys-
Pro-Pro-Gly-Val-Trp-Pro-Ala-Asn-Pro-Ala-Pro-Ile-Thr-Gln-Thr-Val-
Ile-His-Thr-Val-Gln-Ser-Ala-Pro-Gly-Gln-Met-Phe-Ser
(SEQ ID NO: 4), and
Asn-Pro-Arg-Thr-Val-Pro-Val-Gln-Pro-Ala-Phe-Ser-Thr-Val-Pro-Phe-
Ser-Gln-Pro-Val-Cys-Phe-Pro-Pro-Arg-Pro-Arg-Gly-Arg-Arg-Gln-Lys-
Pro-Pro-Gly-Val-Trp-Pro-Ala-Asn-Pro-Ala-Pro-Ile
(SEQ ID NO: 9).

### (b) Preparation of antigen polypeptide

Characteristic sequences are selected from the human galectin-9 amino acid sequence of SEQ ID NO: 1 and these sequences are synthesized. Besides the C terminal CRD of Example 1, peptides are synthesized by the Fmoc-bop method with a peptide synthesizer (Peptide Synthesizer 9600, Milligen/Biosearch). Cysteine is incorporated into the N terminal of the polypeptides. The synthesized peptides are purified by high performance liquid chromatography on a µ Bondasphere C18 column (Waters), etc.

### (c) Preparation of BSA-polypeptide conjugates

Each peptide is coupled with bovine serum albumin (BSA) via a cysteine residue to form an antigen-conjugate. BSA (10.1 mg) is dissolved in 1 mL of 0.1 M phosphate buffer, pH 7.0. Also, 1.14 mg of EMCS (N-(ε -maleimidecaproyloxy)-succinimide) is dissolved in 24.9 µ L of dimethylformamide. A mixture of the BSA solution and the EMCS solution is reacted at 30°C for 30 min., and then subjected to gel filtration through a Sephadex G-25 (Pharmacia) column (13 mm φ × 120 mm long) equilibrated with a 0.1 M phosphate buffer, pH 7.0. Each synthetic polypeptide obtained in the above (b) is dissolved in a 0.1 M phosphate buffer, pH 7.0, then mixed with the maleimido-coupled BSA thus prepared (molar ratio of polypeptide: maleimido-coupled BSA = 50: 1). For example, the polypeptide is mixed with the maleimido-coupled BSA, and incubated at 4°C for 20 hours to form a BSA-polypeptide conjugate. The resultant BSA-polypeptide conjugate is diluted with a 0.1 M phosphate buffer, pH 7.0, poured to each tube (150µ L per tube) and cryopreserved at -30°C.

### (d) Preparation of antibody-producing cells

The BSA-polypeptide conjugate (prepared in the above (b) step) is intraperitoneally administered together with comple te Freund's adjuvants to an 8-week-old female Balb/c mouse for initial immunization. Approximately 18 days later, the BSA-polypeptide conjugate, dissolved in 0.1M phosphate buffer, pH 7.5, is administered intraperitoneally to the primarily immunized mouse for booster. Further approximately 52 days later, the BSA-polypeptide conjugate, dissolved in 0.1M phosphate buffer, pH 7.5 is administered intravenously or intraperitoneally to to the mouse for final immunization. Next four days later, the spleen is taken out, and the spleen cell suspension is prepared. Immunization may also be carried out with the C-terminal CRD of Example 1 in the same manner.

### (e) Cell fusion

For cell fusion, the following materials and methods are used:
RPMI-1640 or hybridoma culture medium:
   To RPMI-1640 is added sodium bicarbonate (24 mM), sodium pyruvate (1 mM), and an antibiotic (selected from penicillin G potassium (50 U/ml), amikacin sulfate (100µ g/ml), streptomycin (10 µ g/ml), and amphotericin B (0.25 µ g/ml)), and the mixture is adjusted to pH 7.2 with dry ice, sterilized and filtered through a 0.2µ m Toyo Membrane Filter. Commercially available hybridoma culture media may be used instead.
NS-1 medium:
   To the above RPMI-1640 medium is added filter-sterilized fetal calf serum (FCS, M. A. Bioproducts) until a concentration of FCS reaches 15% (v/v).
PEG 4000 solution:
   To RPMI-1640 medium is added polyethylene glycol 4000 (PEG-4000, Merck & Co.) until a concentration of PEG 4000 reaches 50% (w/w). Thus, the serum-free solution is prepared.

Cell fusion using 8-azaguanine-resistant myeloma cells P3U1 (P3× 63-Ag.8U.1) or × 63 (P3× 63Ag8) is carried out by slightly modified methods according to Oi et al. techniques disclosed in "Selected Method in cellular immunology", pp.351 to 372 (ed. B. B. Mishell and S. N. Shiigi), W. H. Freeman and Company (1980).

The respective nucleated spleen cells (viable cell ratio: 100%) prepared in the foregoing (d) are fused with myeloma cells (viable cell ratio: 100%) in a ratio of approximately 5:1 to 10:1 according to the following procedure:

Each polypeptide-immunized spleen cell suspension and the myeloma cells are washed respectively with a RPMI-1640 medium followed by resuspending in the same medium. For fusion, the nucleated spleen cells and the myeloma cells are mixed together. That is, approximately 4.0 x 10^{a} nucleated spleen cells are mixed together with approximately 8.0 x 10⁷ myeloma cells.

The cell suspension is then precipitated by centrifugation and the supernatant is completely aspirated off. To the cell pellet is added a PEG 4000 solution (RPMI-1640 medium containing 50% polyethylene glycol 4000) pre-warmed to 37°C dropwise (the volume of the PEG 4000 solution to be added is determined to give about 3 x 10⁷ myeloma cells/mL), and stirred to allow the cells to be resuspended and dispersed. Next, after 37°C pre-warmed RPMI 1640 medium is added dropwise (RPMI-1640 medium : 50% PEG 4000-containing RPMI 1640 medium already added = 2:1 in volume), the same medium is added dropwise with constantly stirring (RPMI-1640 medium : 50% PEG 4000-containing RPMI 1640 medium = 7:1 in volume) to allow the cells to be dispersed. This cell dispersion is centrifuged, and the supernatant fluid is completely aspirated off. To the cell pellet is added 37°C pre-warmed NS-1 medium quickly until a concentration of myeloma cells reaches approximately 3 x 10⁶ cells/mL, and a large cell mass is carefully dispersed by pipetting. Next, the cell suspension is diluted with the same medium, and 6.0 x 10⁵ cells/well is plated on each well of a polystyrene 96-well microplate. The cell-containing microwell is incubated at 37°C in an atmosphere containing 7% CO₂/93% air and with 100% relative humidity.

### (f) Selective growth of hybridomas in selection medium

### (1) Media to be used are as follows:

HAT medium: To the RPMI-1640 or hybridoma culture medium as described in the above (e) is added further hypoxanthine (100µ M), aminopterin (0.4µ M), and thymidine (16 µ M).
HT medium: The medium has the same composition as the above HAT medium except that aminopterin is excluded.

(2) Next day (1st day) from culture initiation of the foregoing (e), two drops (approximately 0.1mL) of HAT medium is added to the cells with a Pasteur pipette. On the 2nd, 3rd, 5th, and 8th days, half of the medium (approximately 0.1 mL) is replaced with fresh HAT medium, respectively. On the 10th day, half of the medium is replaced with fresh HT medium. All wells wherein the growth of hybridomas is visually recognized are examined for positive wells by solid phase-antibody binding tests (enzyme-linked immunosorbent assay; ELISA). First, polystyrene 96-well plates are coated with each antigen polypeptide (100ng/well) wherein the polypeptide is diluted with a 20 mM carbonate buffer, pH 9.6, and washed with PBS containing 0.05% Tween 20 for washing to remove unadsorbed peptides. To each polypeptide-coated well is added an aliquot (0.1 ml) of supernatant fluid from the hybridoma well in which hybridoma growth is recognized and the polypeptide-coated well is allowed to stand at room temperature for approximately one hour. After washing, horseradish peroxidase (HRP)-labeled goat anti-mouse immunoglobulin (Cappel Lab.) is added as a second antibody to the polypeptide-coated well, and the well is further allowed to stand at room temperature for approximately 1 hour. Next, after washing, to the well is added substrates, hydrogen peroxide and 3,3',5,5'-tetramethylbenzidine (TMB), leading to coloring. The coloration reaction is then stopped by adding 2N sulfuric acid to each well and OD readings at 450 nm are obtained by a microplate OD reader (MRP-A4, Toso). The above procedures may also be carried out with recombinant protein antigens.

### (g) Cloning of hybridomas

Hybridomas in the positive well (against each antigen peptide or protein) obtained in the foregoing (f) are cloned by limiting dilution to establish monoclones. That is, a cloning medium containing, as feeder cells, approximately 10⁷ mouse thymocytes per 1 ml of NS-1 medium is prepared. Into a 96-well microplate are plated hybridomas at a cell density of 5, 1, or 0.5 cells per well, respectively, with dilutions wherein the 5, 1, or 0.5 hybridoma cells per well is plated to 36, 36, and 24 wells, respectively. On the 5th and 12th days, an aliquot (about 0.1 ml) of NS-1 medium is added to all the wells. ELISA as described in the above (f) is conducted for groups wherein the sufficient growth of hybridomas is visually recognized and the rate of colony formation-negative wells is 50% or more. In cases where all the examined wells are negative, 4 to 6 wells each containing 1 colony are selected from antibody-positive wells, and recloned. Finally, anti-target polypeptide antibody-producing hybridoma cells are obtained.

### (h) Determination of class and subclass for monoclonal antibodies

According to ELISA as described herein above, each supernatant obtained in the above (g) is added to polystyrene 96-well plates coated with each polypeptide. Next, after PBS washing, iso-type specific rabbit anti-mouse IgG antibodies (Zymed Lab.) are added. After PBS washing, horseradish peroxidase-labeled goat anti-rabbit IgG (H+L) is added, and visualization is carried out with hydrogen peroxide and 2,2'-azino-di(3-ethylbenzothiazolinic acid). As a result, the class and sub-class are determined. These procedures can likewise be carried out using recombinant protein antigens.

### (i) Hybridoma cultivation and purification of monoclonal antibodies

Each hybridoma cell thus obtained is grown in NS-1 medium to afford monoclonal antibodies in the culture supernatant. Alternatively, 10⁷ hybridoma cells thus obtained are administered intraperitoneally to a mouse (inbred Balb/c mouse, female, 6-week old) primed intraperitoneally with Pristane (2,6,10,14-tetramethyl pentadecane) 1 week prior to hybridoma injection, and 1 to 2 weeks later ascites containing 4 to 7 mg/ml monoclonal antibody may be recollected. The obtained ascites are salted out with 40% ammonium sulfate saturation, IgG class antibodies are adsorbed on protein A affigel (Bio-Rad), followed by elution with 0.1 M citrate buffer, pH 5.0 to afford purified forms.

### Example 6

### Sandwich EIA

Sandwich EIA systems capable of specifically detecting and/or measuring human galectin-9 can be constructed by a combination of suitable two antibodies selected from anti-human galectin-9 antibodies as prepared in Example 1, and monoclonal anti-human galectin-9 antibodies as prepared in Example 5, according to techniques as disclosed herein below. The EIA systems may include either of one-step and two-step techniques but labeled antibodies are not limited to Fab'-HRP. Compositions for each reaction buffer, reaction conditions can be adjusted depending on various purposes of assaying. For example, the reaction time can be shortened or extended. Standard human galectin-9 samples can be obtained by isolation and purification from tissue culture supernatants, cell culture supernatants, and transformants or transfectants wherein the human galectin-9 is expressed by techniques as described in Example 1 or other methods. The purification can be conducted by a combination of ion-exchange chromatography, gel filtration, affinity chromatography using monoclonal anti-human galectin-9 antibodies, and/or other various affinity chromatography.

### (a) Preparation of labeled antibodies

Monoclonal anti-human galectin-9 antibodies are digested with pepsin (enzyme: antibody ratio, 2% w/w) in 0.1M acetate buffer, pH4.2 containing 0.1M NaCl at 37°C for 24 hrs. The digestion is stopped with the addition of 3M Tris-HCl buffer, pH7.5. F(ab')₂ fractions are collected by gel filtration on an Ultrogel AcA54 column equilibrated with 0.1M phosphate buffer, pH7.0. Cysteamine hydrochloride is then added to the F(ab')₂ fractions until the final concentration reaches to 0.01M. The fragments are reduced at 37°C for 1.5 hr. Fab' fractions are collected by gel filtration on an Ultrogel AcA54 column equilibrated with 0.1M phosphate buffer, pH6.0 containing 5mM EDTA.

In another procedure, HRP is dissolved in 0.1 M phosphate buffer, pH7.0, followed by addition of EMCS in DMF (EMCS:HRP molar ratio, 25:1). The mixture is incubated at 30°C for 30 min. and subjected to gel filtration on an NICK-5 column (Pharmacia) equilibrated with 0.1 M phosphate buffer, pH6.0 to collect maleimide-conjugated HRP fractions.

The Fab' fraction is mixed with the maleimide-conjugated HRP fraction to form an equimolar mixture. The resultant mixture is incubated at 4°C for 20 hrs, followed by blocking of unreacted thiol groups with N-ethyl maleimide (N-ethyl maleimide:Fab' molar ratio, 10:1). The mixture is subjected to gel filtration on an Ultrogel AcA54 column equilibrated with 0.1M phosphate buffer, pH6.5 to collect labeled antibodies, Fab'-HRP conjugates. The labeled antibody fraction supplemented with 0.1% BSA and 0.001% chlorhexidine is stored at 4°C. Similarly, the process may be carried out with anti-human galectin-9 antibody as disclosed in Example 1.

### (b) Preparation of monoclonal antibody-coated carriers

Monoclonal anti-human galectin-9 antibodies are dissolved in 0.1M phosphate buffer, pH 7.5 to a concentration of 50 µ g/mL. The monoclonal antibody solution is added to each well of a 96-well microplate at 100µ L per well and allowed to stand at 4°C for 18 hrs. After removal of the monoclonal antibody solution, each well is rinsed once with a physiological saline and three times with Tris-HCl buffer, pH 8.0 containing 0.05% Tween 20, 0.1M NaCl, and 5mM CaCl₂, and blocked by the addition of Tris-HCl buffer, pH8.0 containing 1% BSA, 0.1M NaCl and 5mM CaCl₂. The anti-human galectin-9 antibody of Example 1 may be used and treated in the same manner to form a solid-phase antibody.

### (c) One-step sandwich EIA method

With a purified human galectin-9 fraction as a standard antigen is prepared a standard curve for quantitative assay of human galectin-9. The standard human galectin-9 samples diluted serially with Tris-HCl buffer, pH 8.0, containing 1% BSA, 0.05% Brij 35, 0.05% Tween 20, 0.1M NaCl, and 5mM CaCl₂, are dispensed into each well, followed by addition of the labeled antibody, Fab'-HRP, which is resuspended in Tris-HCl buffer, pH 8.0 containing 1% BSA, 0.05% Brij 35, 0.05% Tween 20, 0.1M NaCl and 5mM CaCl₂. The samples are well mixed. The antibody-bound microplates prepared herein above are rinsed three times with Tris-HCl buffer, pH8.0 containing 0.05% Tween 20, 0.1M NaCl, and 5mM CaCl₂. To each well is added the standard antigen-labeled antibody mixture. After incubation at room temperature for 1 hr, each well is rinsed three times with Tris-HCl buffer, pH8.0 containing 0.05% Tween 20, 0.1M NaCl, and 5mM CaCl₂. Next, to each well is added a 0.01% solution of 3,3',5,5'-tetramethylbenzidine in 0.1M acetate buffer (pH 5.5) containing 6% dimethylformamide and 0.005% hydrogen peroxide and incubated at room temperature for 20 min. The reaction is stopped with the addition of 2N sulfuric acid. The reaction mixtures are read at 492 nm in a microplate reader to prepare a standard curve.

Samples to be measured or assayed may be prepared from human body fluids including human serum, spinal fluid, plasma, articular fluid, urine, saliva, etc., various human tissue extracts, various cultured cell extracts and culture supernatants derived from human origins or recombinants, etc. Each sample to be measured or assayed may be subjected to one-step sandwich EIA as disclosed herein above in place of the standard human galectin-9 sample. The reaction for the sample to be measured or assayed is conducted simultaneously with that for the standard human galectin-9 sample. The absorbance values obtained from tested samples are analyzed by reference to the standard binding curve to estimate an amount of human galectin-9 contained in the tested samples.

Besides the above, the anti-human galectin-9 antibody of Example 1 may be used to prepare enzyme-labeled antibodies which can be applied to measurements, detections and/or assays in accordance to the methods as disclosed in Ikuzo Uriya et al. ed., "Seibutsukagaku Jikkenho 27" (Biological Chemistry Laboratory Techniques 27): Eiji Ishikawa, "Koso Hyoshikiho" (Enzyme Labeling Methods), Gakkai Shuppan Center Co., Ltd., Japan (June 20, 1991) and The Japanese Biochemical Society ed., "Zoku Seikagaku Jikken Koza 5, Menekiseikagaku Kenkyuho" (Supplementary Lectures on Biochemical Laboratory Techniques 5, Immunobiochemistry Research Methods), Tokyo Kagaku Dojin, Japan (March 14, 1986) (including methods as indicated in documents quoted therein).

### Example 7

### Western blotting

A culture supernatant of human galectin-9-expressing cells, purified recombinant human galectin-9, and human melanoma-derived protein extracts were subjected to 10 to 15% SDS-PAGE separation under reducting conditions and then transferred onto polyvinylidene difluoride (PVDF) membranes (MILLIPORE). The membranes were blocked in TBS (blocking buffer), containing 5% BSA or 5% skim milk and 0.05% sodium azide at room temperature for 0.5 to 1 hr, treated with human galectin-9-specific antibody, and incubated at 25°C for 6 hr or less. Each membrane was then washed 4 times with TBS (containing 0.05% sodium azide), containing 0.1% Tween 20, and the bound antibody was reacted with HRP-conjugated donkey anti-rabbit immunoglobulin antibody or HRP-conjugated rabbit anti-mouse immunoglobulin antibody (the antibody was diluted with the the blocking buffer at 1:1,000) at 25°C for 1 hour. After reaction, each membrane was washed 4 times with TBS (containing 0.05% sodium azide), containing 0.1% Tween 20, and the bound antibody is detected by enhanced chemiluminescence (ECL, Amersham Pharmacia).

Besides the above, the anti-human-galectin-9 antibody of Example 1 may be used to perform Western blotting according to the methods as disclosed in Yoshiyuki Kuchino, et al. ed., "Idenshi·Tanpakushitsu, Jikken Sosa Burottingu-ho" (Gene and Protein, Experimental Procedures, Blotting Methods), Soft Science Co., Ltd., Japan, Nov. 10, 1987 (including methods as indicated in documents quoted therein).

### Industrial Applicability

As has been described in detail above herein, the present invention provides cancer metastatic potential-detecting agents capable of detecting or assessing whether or not cancer cells have metastatic potential and/or how much metastatic level such cancer cells have. Since the cancer metastasis detecting agents according to the present invention contains anti-galectin-9 Ab as an effective component, it enables the detection of the metastatic potential owned by cancer cells in an extremely simple and yet definite manner. The present invention also enables the arrangement of galectin-9 expression gene-measuring systems capable of detecting or assessing the metastatic potential of cancer cells in an extremely simple and yet definite fashion.

Furthermore according to the present invention, the use of galectin-9 and a galectin-9 expression gene as a cancer metastatic potential marker or tumor marker enables the arrangement of cancer metastatic potential-detection agents or tumor detection and/or measurement agents, cancer metastatic potential detection methods (assays) or tumor detection and/or measurement methods (assays), and cancer metastatic detection or tumor detection and/or measurement reagent sets or various forms of systems that are not only helpful to but are also excellent for the diagnosis, prevention, and therapy of cancers.

While the present invention has been described specifically in detail with reference to certain embodiments and examples thereof, it would be apparent that it is possible to practice it in other forms. In light of the disclosure, it will be understood that various modifications and variations are within the spirit and scope of the appended claims.

## Claims

1. A cancer metastatic potential prediction and/or detection agent comprising an effective amount of an anti-galectin-9 antibody against galectin-9.

2. The cancer metastatic potential prediction and/or detection agent as set forth in Claim 1, wherein said cancer is selected from the group consisting of malignant tumors of organs, tissues, and blood, including epithelial malignant tumors and non-epithelial malignant tumors (including those that are tumorigenic and non-tumorigenic).

3. The cancer metastatic potential prediction and/or detection agent as set forth in Claim 1 or 2, wherein said cancer is selected from the group consisting of at least skin cancer (which may include melanoma), mammary cancer, ovarian cancer, uterine cancer, malignant testicular tumor, prostatic cancer, urinary bladder cancer, renal cancer, thyroid cancer, pharyngeal/larynx cancer, lingual cancer, maxillary cancer, esophageal cancer, stomach cancer, colon/rectal cancer, lung/bronchial cancer, liver cancer (including liver cell cancer and intrahepatic bile duct cancer), extrahepatic bile duct/gall bladder cancer, pancreatic cancer, leukemia, malignant lymphoma, plasmacytoma, osteosarcoma, chondrosarcoma, leiomyosarcoma, rhabdomyosarcoma, liposarcoma, fibrosarcoma, malignant hemangioma, malignant hemangioendothelioma, brain tumor (including meningioma, glyoma, astrocytoma), and other cancers.

4. The cancer metastatic potential prediction and/or detection agent as set forth in any of Claims 1 through 3, wherein said cancer cells are mammary cancer cells or melanoma cells.

5. The cancer metastatic potential prediction and/or detection agent as set forth in any of Claims 1 through 4, with which galectin-9 in a test sample is quantitatively assayed using an immunological method and cancer metastatic potential is detected or assessed using the result of this quantitative assay.

6. The cancer metastatic potential prediction and/or detection agent as set forth in any of Claims 1 through 5, wherein an immunological method is selected from the group consisting of immunostaining, including tissue and cell staining, immunoelectron microscopy, and immunoassays, including RIA, EIA, FIA, LIA, and sandwich assays as well as competitive immunoassays and non-competitive immunoassays.

7. The cancer metastatic potential prediction and/or detection agent as set forth in any of Claims 1 through 6, comprising an effective amount of an immobilized anti-galectin-9 antibody.

8. The cancer metastatic potential prediction and/or detection agent as set forth in any of Claims 1 through 6, comprising an effective amount of a labeled anti-galectin-9 antibody.

9. A cancer metastatic potential prediction and/or detection agent comprising an effective amount of a nucleic acid, which hybridizes with a nucleic acid coding for galectin-9 or a constituent domain thereof.

10. The cancer metastatic potential prediction and/or detection agent as set forth in Claim 9, wherein said nucleic acid is a probe, which may be labeled and which hybridizes with a nucleic acid coding for galectin-9 or a constituent domain thereof.

11. The cancer metastatic potential prediction and/or detection agent as set forth in Claim 9, wherein said nucleic acid is a primer, with which, by polymerase chain reaction (PCR), a nucleic acid coding for galectin-9 or a constituent domain thereof can be amplified.

12. The cancer metastatic potential prediction and/or detection agent as set forth in any of Claims 9 through 11, with which a galectin-9 expression gene is measured and/or detected by a method selected from the group consisting of in situ hybridization, Northern blotting, dot blotting, RNase protection assay, RT-PCR, real-time PCR, and DNA array analysis.

13. A cancer metastatic potential prediction and/or detection method using the cancer metastatic potential prediction and/or detection agent as set forth in any of Claims 1 through 12 to measure or assess a biological sample.

14. A cancer metastatic potential prediction and/or detection of measuring, detecting, or quantitatively assaying for galectin-9 or a galectin-9 expression gene in a test sample and using the result to detect or assess cancer metastatic potential.

15. The cancer metastatic potential prediction and/or detection as set forth in Claim 13 or 14, wherein the expression amount of galectin-9 or the amount of a galectin-9 expression gene in a test sample is measured, the expression amount or amount of expression gene obtained by said measurement is compared to judge whether or not it falls below a fixed threshold value, and said measurement of galectin-9 expression amount or expression gene amount is carried out by the use of (1) a composition, comprising an effective amount of an anti-galectin-9 antibody against galectin-9; or (2) a composition, comprising an effective amount of a nucleic acid, which hybridizes with a nucleic acid coding for galectin-9 or a constituent domain thereof.

16. A reagent set or system to be used in the prediction and/or detection as set forth in any of Claims 13 to 15, said reagent set or system comprising (1) an effective amount of an anti-galectin-9 antibody against galectin-9; or (2) an effective amount of a nucleic acid, which hybridizes with a nucleic acid coding for galectin-9 or a constituent domain thereof.

17. A cancer metastatic potential prediction and/or detection agent or tumor detection and/or measurement agent, in which galectin-9 or a galectin-9 expression gene is employed as a cancer metastatic potential marker or tumor marker.

18. The cancer metastatic potential prediction and/or detection agent or tumor detection and/or measurement agent as set forth in Claim 17, wherein galcetin-9 or a galectin-9 expression gene is employed as a prognostic cancer metastatic potential marker or tumor marker.

19. A cancer metastatic potential prediction and/or detection method or tumor detection and/or measurement, in which galectin-9 or a galectin-9 expression gene is employed as a cancer metastatic potential marker or tumor marker.

20. The cancer metastatic potential prediction and/or detection method or tumor detection and/or measurement as set forth in Claim 19, wherein galcetin-9 or a galectin-9 expression gene is employed as a prognostic cancer metastatic potential marker or tumor marker.

21. A cancer metastatic potential prediction and/or detection or tumor detection and/or measurement reagent set or system, in which galectin-9 and a galectin-9 expression gene is employed as a cancer metastatic potential marker or tumor marker.

22. The cancer metastatic potential prediction and/or detection or tumor detection and/or measurement reagent set or system as set forth in Claim 21, wherein galcetin-9 or a galectin-9 expression gene is employed as a prognostic cancer metastatic potential marker or tumor marker.


SEQUENCE LISTING

<110> GALPHARMA Co., Ltd.

<120> A detective agent for a metastasis

<130> GL-01PCT

<140>
<141>

<150> JP 2000-335077
<151> 2000-11-01

<160> 9

<170> PatentIn Ver. 2.0

<210> 1
<211> 355
<212> PRT
<213> Homo sapiens

<400> 1
Met Ala Phe Ser Gly Ser Gln Ala Pro Tyr Leu Ser Pro Ala Val Pro
  1               5                  10                  15

Phe Ser Gly Thr Ile Gln Gly Gly Leu Gln Asp Gly Leu Gln Ile Thr
             20                  25                  30

Val Asn Gly Thr Val Leu Ser Ser Ser Gly Thr Arg Phe Ala Val Asn
         35                  40                  45

Phe Gln Thr Gly Phe Ser Gly Asn Asp Ile Ala Phe His Phe Asn Pro
     50                  55                  60

Arg Phe Glu Asp Gly Gly Tyr Val Val Cys Asn Thr Arg Gln Asn Gly
 65                  70                  75                  80

Ser Trp Gly Pro Glu Glu Arg Lys Thr His Met Pro Phe Gln Lys Gly
                 85                  90                  95

Met Pro Phe Asp Leu Cys Phe Leu Val Gln Ser Ser Asp Phe Lys Val
            100                 105                 110

Met Val Asn Gly Ile Leu Phe Val Gln Tyr Phe His Arg Val Pro Phe
        115                 120                 125

His Arg Val Asp Thr Ile Ser Val Asn Gly Ser Val Gln Leu Ser Tyr
    130                 135                 140

Ile Ser Phe Gln Asn Pro Arg Thr Val Pro Val Gln Pro Ala Phe Ser
145                 150                 155                 160

Thr Val Pro Phe Ser Gln Pro Val Cys Phe Pro Pro Arg Pro Arg Gly
                165                 170                 175

Arg Arg Gln Lys Pro Pro Gly Val Trp Pro Ala Asn Pro Ala Pro Ile
            180                 185                 190

Thr Gln Thr Val Ile His Thr Val Gln Ser Ala Pro Gly Gln Met Phe
        195                 200                 205

Ser Thr Pro Ala Ile Pro Pro Met Met Tyr Pro His Pro Ala Tyr Pro
    210                 215                 220

Met Pro Phe Ile Thr Thr Ile Leu Gly Gly Leu Tyr Pro Ser Lys Ser

225                 230                 235                 240

Ile Leu Leu Ser Gly Thr Val Leu Pro Ser Ala Gln Arg Phe His Ile
                245                 250                 255

Asn Leu Cys Ser Gly Asn His Ile Ala Phe His Leu Asn Pro Arg Phe
            260                 265                 270

Asp Glu Asn Ala Val Val Arg Asn Thr Gln Ile Asp Asn Ser Trp Gly
        275                 280                 285

Ser Glu Glu Arg Ser Leu Pro Arg Lys Met Pro Phe Val Arg Gly Gln
    290                 295                 300

Ser Phe Ser Val Trp Ile Leu Cys Glu Ala His Cys Leu Lys Val Ala
305                 310                 315                 320

Val Asp Gly Gln His Leu Phe Glu Tyr Tyr His Arg Leu Arg Asn Leu
                325                 330                 335

Pro Thr Ile Asn Arg Leu Glu Val Gly Gly Asp Ile Gln Leu Thr His
            340                 345                 350

Val Gln Thr
        355


<210> 2
<211> 323
<212> PRT
<213> Homo sapiens

<400> 2

<210> 3
<211> 311
<212> PRT
<213> Homo sapiens

<400> 3
Met Ala Phe Ser Ser Ser Gln Ala Pro Tyr Leu Ser Pro Ala Val Pro
1               5                   10                  15

Phe Ser Gly Thr Ile Gln Gly Gly Leu Gln Asp Gly Leu Gln Ile Thr
            20                  25                  30

Val Asn Gly Thr Val Leu Ser Ser Ser Gly Thr Arg Phe Ala Val Asn
        35                  40                  45

Phe Gln Thr Gly Phe Ser Gly Asn Asp Ile Ala Phe His Phe Asn Pro
    50                  55                  60

Arg Phe Glu Asp Gly Gly Tyr Val Val Cys Asn Thr Arg Gln Asn Gly
65                  70                  75                  80

Ser Trp Gly Pro Glu Glu Arg Lys Thr His Met Pro Phe Gln Lys Gly
                85                  90                  95

Met Pro Phe Asp Leu Cys Phe Leu Val Gln Ser Ser Asp Phe Lys Val
            100                 105                 110

Met Val Asn Gly Ile Leu Phe Val Gln Tyr Phe His Arg Val Pro Phe
        115                 120                 125

His Arg Val Asp Thr Ile Ser Val Asn Gly Ser Val Gln Leu Ser Tyr
    130                 135                 140

Ile Ser Phe Gln Thr Gln Thr Val Ile His Thr Val Gln Ser Ala Pro
145                 150                 155                 160

Gly Gln Met Phe Ser Thr Pro Ala Ile Pro Pro Met Met Tyr Pro His

165                 170                 175

Pro Ala Tyr Pro Met Pro Phe Ile Thr Thr Ile Leu Gly Gly Leu Tyr
            180                 185                 190

Pro Ser Lys Ser Ile Leu Leu Ser Gly Thr Val Leu Pro Ser Ala Gln
        195                 200                 205

Arg Phe His Ile Asn Leu Cys Ser Gly Asn His Ile Ala Phe His Leu
    210                 215                 220

Asn Pro Arg Phe Asp Glu Asn Ala Val Val Arg Asn Thr Gln Ile Asp
225                 230                 235                 240

Asn Ser Trp Gly Ser Glu Glu Arg Ser Leu Pro Arg Lys Met Pro Phe
                245                 250                 255

Val Arg Gly Gln Ser Phe Ser Val Trp Ile Leu Cys Glu Ala His Cys
            260                 265                 270

Leu Lys Val Ala Val Asp Gly Gln His Leu Phe Glu Tyr Tyr His Arg
        275                 280                 285

Leu Arg Asn Leu Pro Thr Ile Asn Arg Leu Glu Val Gly Gly Asp Ile
    290                 295                 300

Gln Leu Thr His Val Gln Thr
305                 310


<210> 4
<211> 61
<212> PRT
<213> Homo sapiens

<400> 4
Asn Pro Arg Thr Val Pro Val Gln Pro Ala Phe Ser Thr Val Pro Phe
1               5                   10                  15

Ser Gln Pro Val Cys Phe Pro Pro Arg Pro Arg Gly Arg Arg Gln Lys
            20                  25                  30

Pro Pro Gly Val Trp Pro Ala Asn Pro Ala Pro Ile Thr Gln Thr Val
        35                  40                  45

Ile His Thr Val Gln Ser Ala Pro Gly Gln Met Phe Ser
    50                  55                  60


<210> 5
<211> 29
<212> PRT
<213> Homo sapiens

<400> 5
Pro Pro Gly Val Trp Pro Ala Asn Pro Ala Pro Ile Thr Gln Thr Val
1               5                   10                  15

Ile His Thr Val Gln Ser Ala Pro Gly Gln Met Phe Ser
            20                  25


<210> 6
<211> 17
<212> PRT
<213> Homo sapiens

<400> 6
Thr Gln Thr Val Ile His Thr Val Gln Ser Ala Pro Gly Gln Met Phe
1               5                   10                  15

Ser

<210> 7
<211> 32
<212> PRT
<213> Homo sapiens

<400> 7
Asn Pro Arg Thr Val Pro Val Gln Pro Ala Phe Ser Thr Val Pro Phe
1               5                   10                  15

Ser Gln Pro Val Cys Phe Pro Pro Arg Pro Arg Gly Arg Arg Gln Lys
            20                  25                  30

<210> 8
<211> 12
<212> PRT
<213> Homo sapiens

<400> 8
Pro Pro Gly Val Trp Pro Ala Asn Pro Ala Pro Ile
1               5                   10

<210> 9
<211> 44
<212> PRT
<213> Homo sapiens

<400> 9
Asn Pro Arg Thr Val Pro Val Gln Pro Ala Phe Ser Thr Val Pro Phe
1               5                   10                  15

Ser Gln Pro Val Cys Phe Pro Pro Arg Pro Arg Gly Arg Arg Gln Lys
            20                  25                  30

Pro Pro Gly Val Trp Pro Ala Asn Pro Ala Pro Ile
        35                  40